# EUROPEAN PATENT SPECIFICATION

(11) **EP 2 297 286 B1**
(45) Date of publication and mention of the grant of the patent: **28.12.2011**
(21) Application number: 09759188.7
(22) Date of filing: 01.06.2009
(51) Int. Cl.: C11B 1/00, C11C 1/00, C10L 1/02, C12P 7/64, C12P 19/02

(54) **A METHOD OF PRODUCING FATTY ACIDS FOR BIOFUEL, BIODIESEL, AND OTHER VALUABLE CHEMICALS**
VERFAHREN ZUR HERSTELLUNG VON FETTSÄUREN FÜR BIOBRENNSTOFF, BIODIESEL UND ANDERE WERTVOLLE CHEMIKALIEN
PROCÉDÉ DE PRODUCTION D'ACIDES GRAS POUR UN BIOCARBURANT, UN BIODIESEL ET D'AUTRES PRODUITS CHIMIQUES DE VALEUR

(30) Priority: 02.06.2008 US 58096
(43) Date of publication of application: 23.03.2011
(73) Proprietor: De Crecy, Eudes, Gainesville, FL 32608 (US)
(72) Inventor: De Crecy, Eudes, Gainesville, FL 32608 (US)
(74) Representative: Loyer & Abello
(86) International application number: PCT/US2009/045882
(87) International publication number: WO 2009/149027

(56) References cited:
- US-A- 4 368 056
- CHUNG, Y.-C., BAKALINSKY, A., AND PENNER, M.H.: "Enzymatic saccharification and fermentation of xylose-optimized dilute acid-treated lignocellulosics" APPLIED BIOCHEMISTRY AND BIOTECHNOLOGY, vol. 121-124, 2005, pages 947-962, XP002554724 Humana Press, Inc ISSN: 0273-2289
- DATABASE COMPENDEX [Online] ENGINEERING INFORMATION, INC., NEW YORK, NY, US; 1997, HABIB A D ET AL: "Conversion of paper sludge into land application materials and diesel fuel precursors" XP002554725 Database accession no. EIX97083477481
- FALL R ET AL: "BIO CONVERSION OF XYLAN TO TRI GLYCERIDES BY OIL RICH YEASTS" APPLIED AND ENVIRONMENTAL MICROBIOLOGY, AMERICAN SOCIETY FOR MICROBIOLOGY, US, vol. 47, no. 5, 1 January 1984 (1984-01-01), pages 1130-1134, XP002532684 ISSN: 0099-2240
- DAI CHUAN-CHAO ET AL: "Biodiesel generation from oleaginous yeast Rhodotorula glutinis with xylose assimilating capacity" 1 September 2007 (2007-09-01), AFRICAN JOURNAL OF BIOTECHNOLOGY, ACADEMIC PRESS, US, PAGE(S) 2130 - 2134 , XP002532682 ISSN: 1684-5315 the whole document

## Description

### BACKGROUND OF THE INVENTION

Petroleum is a non-renewable resource. As a result, many people are worried about the eventual depletion of petroleum reserves in the future. World petroleum resources have even been predicted by some to run out by the 21^{st} century (Kerr RA, Science 1998, 281, 1128).

This has fostered the expansion of alternative hydrocarbon products such as ethanol or other microbial fermentation products from plant derived feed stock and waste. In fact, current studies estimate that the United States could easily produce 1 billion dry tons of biomass (biomass feedstock) material (over half of which is waste) per year. This is primarily in the form of cellulosic biomass.

Cellulose is contained in nearly every natural, free-growing plant, tree, and bush, in meadows, forests, and fields all over the world without agricultural effort or cost needed to make it grow.

It is estimated that these cellulosic materials could be used to produce enough ethanol to replace 30% or more of the US energy needs in 2030. The great advantage of this strategy is that cellulose is the most abundant and renewable carbon source on earth and its efficient transformation into a useable fuel could solve the world's energy problem.

Cellulosic ethanol has been researched extensively. Cellulosic ethanol is chemically identical to ethanol from other sources, such as corn starch or sugar, but has the advantage that the cellulosic materials are highly abundant and diverse. However, it differs in that it requires a greater amount of processing to make the sugar monomers available to the microorganisms that are typically used to produce ethanol by fermentation.

Although cellulose is an abundant plant material resource, its rigid structure makes cellulose a difficult starting material to process. As a result, an effective pretreatment is needed to liberate the cellulose from the lignin seal and its crystalline structure so as to render it accessible for a subsequent hydrolysis step. By far, most pretreatments are done through physical or chemical means. In order to achieve higher efficiency, some researchers seek to incorporate both effects.

To date, the available pretreatment techniques include acid hydrolysis, steam explosion, ammonia fiber expansion, alkaline wet oxidation and ozone pretreatment. Besides effective cellulose liberation, an ideal pretreatment has to minimize the formation of degradation products because of their inhibitory effects on subsequent hydrolysis and fermentation processes.

The presence of inhibitors makes it more difficult to produce ethanol. Even though pretreatment by acid hydrolysis is probably the oldest and most studied pretreatment technique, it produces several potent inhibitors including furfural and hydroxymethyl furfural (HMF) which are by far regarded as the most toxic inhibitors present in lignocellulosic hydrolysate.

The cellulose molecules are composed of long chains of sugar molecules of various kinds. In the hydrolysis process, these chains are broken down to free the sugar, before it is fermented for alcohol production.

There are two major cellulose hydrolysis processes: i) a chemical reaction using acids, or an ii) an enzymatic reaction. However, current hydrolysis processes are expensive and inefficient. For example, enzymatic hydrolysis processes require obtaining costly cellulase enzymes from outside suppliers.

A further problem in transforming cellulosic products into ethanol is that up to 50% of the available carbon to carbon dioxide is inherently lost through the fermentation process. In addition, ethanol is more corrosive than gas and diesel. As a result, it requires a distinct distribution infrastructure as well as specifically designed engines. Finally, ethanol is 20-30% less efficient than fossil gas and as ethanol evaporates more easily, a higher percentage is lost along the whole production and distribution process.

A process that could produce biodiesel from cellulose would alleviate the problems associated with ethanol and other biodiesel productions.

Biodiesel obtained from microorganisms (e.g., algae and bacteria) is also non-toxic, biodegradable and free of sulfur. As most of the carbon dioxide released from burning biodiesel is recycled from what was absorbed during the growth of the microorganisms (e.g., algae and bacteria), it is believed that the burning of biodiesel releases less carbon dioxide than from the burning of petroleum, which releases carbon dioxide from a source that has been previously stored within the earth for centuries. Thus, utilizing microorganisms for the production of biodiesel may result in lower greenhouse gases such as carbon dioxide.

Some species of microorganisms are ideally suited for biodiesel production due to their high oil content. Certain microorganisms contain lipids and/or other desirable hydrocarbon compounds as membrane components, storage products, metabolites and sources of energy. The percentages in which the lipids, hydrocarbon compounds and fatty acids are expressed in the microorganism will vary depending on the type of microorganism that is grown. However, some strains have been discovered where up to 90% of their overall mass contain lipids, fatty acids and other desirable hydrocarbon compounds (e.g., Botryococcus).

Algae such as *Chlorela sp.* and *Dunaliella* are a source of fatty acids for biodiesel that has been recognized for a long time. Indeed, these eukaryotic microbes produce a high yield of fatty acids (20-80% of dry weight), and can utilize CO₂ as carbon with a solar energy source.

However, the photosynthetic process is not efficient enough to allow this process to become a cost effective biodiesel source. An alternative was to use the organoheterotrophic properties of Algae and have them grow on carbon sources such as glucose. In these conditions, the fatty acid yield is extremely high and the fatty acids are of a high quality. The rest of the dry weight is mainly constituted of proteins. However, the carbon sources used are too rare and expensive to achieve any commercial viability.

Lipid and other desirable hydrocarbon compound accumulation in microorganisms can occur during periods of environmental stress, including growth under nutrient-deficient conditions. Accordingly, the lipid and fatty acid contents of microorganisms may vary in accordance with culture conditions.

The naturally occurring lipids and other hydrocarbon compounds in these microorganisms can be isolated and transesterified to obtain a biodiesel. The transesterification of a lipid with a monohydric alcohol, in most cases methanol, yields alkyl esters, which are the primary component of biodiesel.

The transesterification reaction of a lipid leads to a biodiesel fuel having a similar fatty acid profile as that of the initial lipid that was used (e.g., the lipid may be obtained from animal or plant sources). As the fatty acid profile of the resulting biodiesel will vary depending on the source of the lipid, the type of alkyl esters that are produced from a transesterification reaction will also vary. As a result, the properties of the biodiesel may also vary depending on the source of the lipid. (e.g., see Schuchardt, et al, TRANSESTERIFICATION OF VEGETABLE OILS: A REVIEW, J. Braz. Chem. Soc., vol. 9, 1, 199-210, 1998 and G. Knothe, FUEL PROCESSING TECHNOLOGY, 86, 1059-1070 (2005), ).

### SUMMARY

The present invention relates to a method for producing fatty acids from biomass, and in particular, a method of producing fatty acids from biomass and for producing a biofuel from said fatty acids Method for producing fatty acids from biomass are known from the teaching of US 4 368 056. In particular, the present invention relates to a method of producing fatty acids, by:
(i) inoculating a mixture of at least one of cellulose, hemicellulose, and lignin with at least one microorganism strain that produces one or more cellulases, hemicellulases and laccase, that hydrolyze at least one of cellulose, hemicellulose and lignin, under conditions to produce at least one of glucose, cellobiose, xylose, mannose, galactose, rhamnose, arabinose or other hemicellulose sugars;
(ii) inhibiting growth of said at least one microorganism strain;
(iii) inoculating the mixture of step (ii) with at least one algae strain that metabolizes said at least one of glucose, cellobiose, xylose, mannose, galactose, rhamnose, arabinose or other hemicellulose sugars, under conditions so that said at least one algae strain produces one or more fatty acids; and
optionally, (iv) recovering said one or more fatty acids from said at least one algae strain.

These and other features of the invention will be further described and exemplified with reference to the drawings and detailed description below.

### BRIEF DESCRIPTION OF THE DRAWINGS

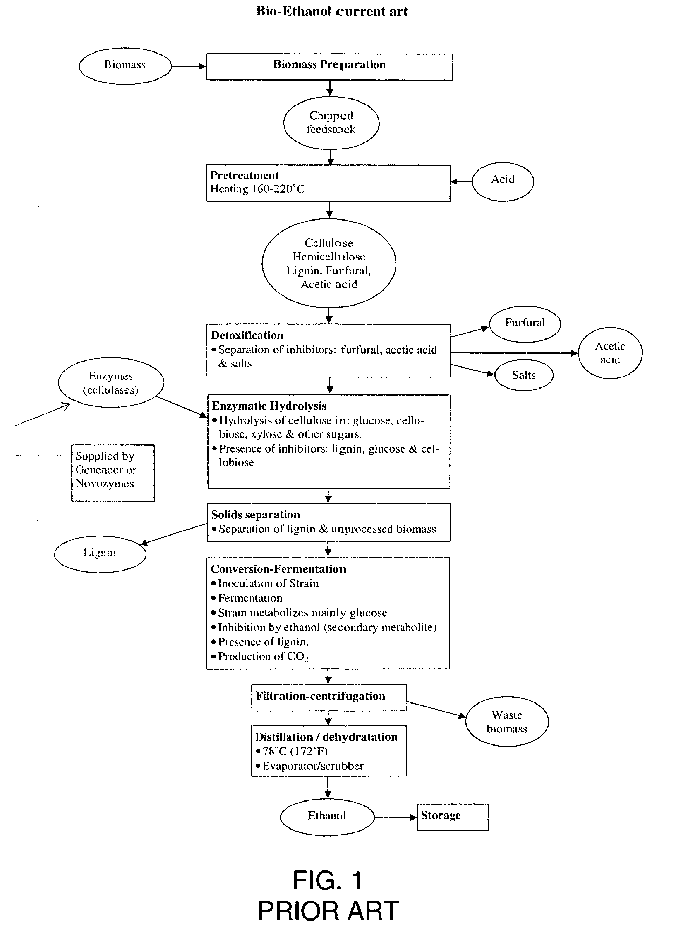

Fig 1. is a flowchart illustrating a conventional process for bio-ethanol production.

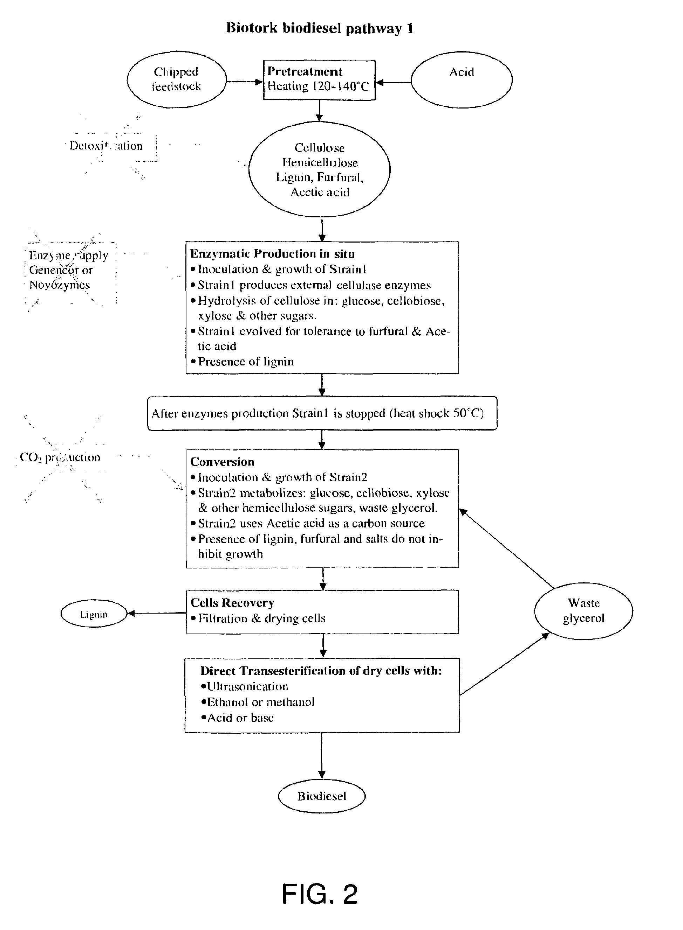

Fig 2. is a flowchart illustrating the general process for fatty acid production and biofuel production of the invention.

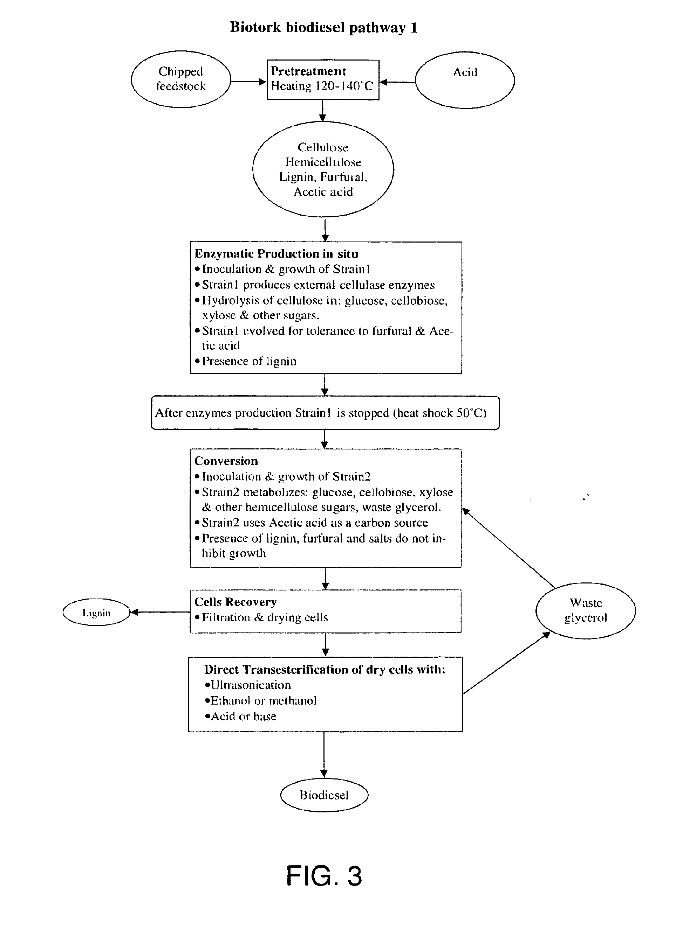

Fig 3. is a flowchart illustrating a specific process for fatty acid production and biofuel production of the invention.
Fig 4. is a flowchart illustrating a preferred embodiment of a specific process for fatty acid production and biofuel production of the invention.

### DETAILED DESCRIPTION OF THE INVENTION

Reference will now be made in detail to embodiments of the invention. Examples of embodiments are illustrated in the accompanying drawings. While the invention will be described in conjunction with these embodiments, it will be understood that it is not intended to limit the invention to such embodiments. On the contrary, it is intended to cover alternatives, modifications, and equivalents as may be included within the spirit and scope of the invention as defined by the appended claims.

In the following description, numerous specific details are set forth in order to provide a thorough understanding of the present invention. The present invention may be practiced without some or all of these specific details. In other instances, well known process operations have not been described in detail in order not to unnecessarily obscure the present invention.

The present invention relates to a method for producing fatty acids from biomass material. The fatty acids can be used, for example, in biofuel production.

One embodiment of the invention is directed to a method of producing fatty acids, by:
(i) inoculating a mixture of at least one of cellulose, hemicellulose, and lignin with at least one microorganism strain that produces one or more cellulases, hemicellulases and laccase, that hydrolyze at least one of cellulose, hemicellulose and lignin, under conditions to produce at least one of glucose, cellobiose, xylose, mannose, galactose, rhamnose, arabinose or other hemicellulose sugars;
(ii) inhibiting growth of said at least one microorganism strain and recovering extracellular and/or intracellular cellulase enzymes in the supernatant (recovery of intracellular cellulase enzyme can be performed by disrupting/breaking cells for release of intracellular enzyme utilizing common techniques, including ultrasonication, French press, temperature, chemical process, enzymatic process, homogenizer, microwaves);
(iii) inoculating the mixture of step (ii) with at least one algae strain that metabolizes said at least one of glucose, cellobiose, xylose, mannose, galactose, rhamnose, arabinose or other hemicellulose sugars, under conditions so that said at least one algae strain produces one or more fatty acids; and
optionally, (iv) recovering said one or more fatty acids from said at least one algae strain.

The mixture in step (i) can be obtained from biomass. Biomass is any organic material made from plants or animals, including living or recently dead biological material, which can be used as fuel or for industrial production. Most commonly, biomass refers to plant matter grown for use as biofuel, but it also includes plant or animal matter used for production of fibers, chemicals or heat. Biomass is a renewable energy source.

There are a wide variety of sources of biomass, including tree and grass crops and forestry, agricultural, and urban wastes, all of which can be utilized in the present invention. Examples of domestic biomass resources include agricultural and forestry residues, municipal solid wastes, industrial wastes, and terrestrial and aquatic crops.

There are many types of plants in the world, and many ways they can be used for energy production. In general there are two approaches: growing plants specifically for energy use, and using the residues from plants that are used for other things. The type of plant utilized in the present invention varies from region to region according to climate, soils, geography, population, and so on.

Energy crops (also called "power crops") can be grown on farms in potentially very large quantities. Trees and grasses, including those native to a region, are preferred energy crops, but other, less agriculturally sustainable crops, including corn can also be used.

Trees are a good renewable source of biomass for processing in the present invention. In addition to growing very fast, certain trees will grow back after being cut off close to the ground (called "coppicing"). This allows trees to be harvested every three to eight years for 20 or 30 years before replanting. Such trees (also called "short-rotation woody crops") grow as much as 40 feet high in the years between harvests. In cooler, wetter regions of the northern United States, varieties of poplar, maple, black locust, and willow are preferred. In the warmer Southeast, sycamore and sweetgum are preferred. While in the warmest parts of Florida and California, eucalyptus and pine are likely to grow well.

Grasses are a good renewable source of biomass for use in the present invention. Thin-stemmed perennial grasses are common throughout the United States. Examples include switchgrass, big bluestem, and other native varieties, which grow quickly in many parts of the country, and can be harvested for up to 10 years before replanting. Thick-stemmed perennials including sugar cane and elephant grass can be grown in hot and wet climates like those of Florida and Hawaii. Annuals, such as corn and sorghum, are another type of grass commonly grown for food.

Oil plants are also a good source of biomass for use in the present invention. Such plants include, for example, soybeans and sunflowers that produce oil, which can be used to make biofuels. Some other oil plants that carry a good yield in oil are poorly used as energy feedstock as their residual bean cake is toxic for mammal nutrition, like jatropha tree or castor bean plant, and are actually good biomass crop. Another different type of oil crop is microalgae. These tiny aquatic plants have the potential to grow extremely fast in the hot, shallow, saline water found in some lakes in the U.S. desert Southwest.

In this regard, biomass is typically obtained from waste products of the forestry, agricultural and manufacturing industries, which generate plant and animal waste in large quantities.

Forestry wastes are currently a large source of heat and electricity, as lumber, pulp, and paper mills use them to power their factories. Another large source of wood waste is tree tops and branches normally left behind in the forest after timber-harvesting operations.

Other sources of wood waste include sawdust and bark from sawmills, shavings produced during the manufacture of furniture, and organic sludge (or "liquor") from pulp and paper mills.

As with the forestry industry, a large volume of crop residue remains in the field after harvest. Such waste could be collected for biofuel production. Animal farms produce many "wet wastes" in the form of manure. Such waste can be collected and used by the present invention to produce fatty acids for biofuel production.

People generate biomass wastes in many forms, including "urban wood waste" (such as shipping pallets and leftover construction wood), the biodegradable portion of garbage (paper, food, leather, yard waste, etc.) and the gas given off by landfills when waste decomposes. Even our sewage can be used as energy; some sewage treatment plants capture the methane given off by sewage and burn it for heat and power, reducing air pollution and emissions of global warming gases.

In one embodiment, the present invention utilizes biomass obtained from plants or animals. Such biomass material can be in any form, including for example, chipped feedstock, plant waste, animal waste, etc.

Such plant biomass typically comprises: about 10-35% lignin; about 15-35% hemicellulose; and about 30-60% cellulose.

The plant biomass that can be utilized in the present invention include at least one member selected from the group consisting of wood, paper, straw, leaves, husks, shells, prunings, grass, including switchgrass, miscanthus, hemp, vegetable pulp, corn, bean cake, corn stover, sugarcane, sugar beets, sorghum, cassava, poplar, willow, potato waste, bagasse, sawdust, and mixed waste of plant, oil palm (palm oil) and forest mill waste.

In one embodiment of the invention, the plant biomass is obtained from at least one plant selected from the group consisting of: switchgrass, corn stover, and mixed waste of plant. In another embodiment, the plant biomass is obtained from switchgrass, due to its high levels of cellulose.

It should be noted that any such biomass material can by utilized in the method of the present invention.

The plant biomass can initially undergo a pretreatment to prepare the mixture utilized in step (i). Pretreatment helps altering the biomass macroscopic and microscopic size and structure, as well as submicroscopic chemical composition and structure, so hydrolysis of the carbohydrate fraction to monomeric sugars can be achieved more rapidly and with greater yields. Common pretreatment procedures are disclosed in Nathan Mosier, Charles Wyman, Bruce Dale, Richard Elander, Y.Y. Lee, Mark Holtzapple, Michael Ladisch, "Features of promising technologies for pretreatment of lignocellulosic biomass," Bioresource Technology: 96, pp. 673-686 (2005), and discussed below.

Pretreatment methods are either physical or chemical. Some methods incorporate both effects (McMillan, 1994; Hsu, 1996). For the purposes of classification, steam and water are excluded from being considered chemical agents for pretreatment since extraneous chemicals are not added to the biomass. Physical pretreatment methods include comminution (mechanical reduction in biomass particulate size), steam explosion, and hydrothermolysis. Comminution, including dry, wet, and vibratory ball milling (Millett et al., 1979; Rivers and Emert, 1987; Sidiras and Koukios, 1989), and compression milling (Tassinari et al., 1980, 1982) is sometimes needed to make material handling easier through subsequent processing steps. Acids or bases could promote hydrolysis and improve the yield of glucose recovery from cellulose by removing hemicelluloses or lignin during pretreatment. Commonly used acid and base include, for example, H₂SO₄ and NaOH, respectively. Cellulose solvents are another type of chemical additive. Solvents that dissolve cellulose in bagasse, cornstalks, tall fescue, and orchard grass resulted in 90% conversion of cellulose to glucose (Ladisch et al., 1978; Hamilton et al., 1984) and showed enzyme hydrolysis could be greatly enhanced when the biomass structure is disrupted before hydrolysis. Alkaline H₂O₂, ozone, organosolv (uses Lewis acids, FeCl₃, (Al)₂SO₄ in aqueous alcohols), glycerol, dioxane, phenol, or ethylene glycol are among solvents known to disrupt cellulose structure and promote hydrolysis (Wood and Saddler, 1988). Concentrated mineral acids (H₂SO₄, HCl), ammonia-based solvents (NH₃, hydrazine), aprotic solvents (DMSO), metal complexes (ferric sodium tartrate, cadoxen, and cuoxan), and wet oxidation also reduce cellulose crystallinity and disrupt the association of lignin with cellulose, as well as dissolve hemicellulose. These methods, while effective, are too expensive for now to be practical when measured against the value of the glucose (approximately 5c/lb). The following pretreatment methods of steam explosion, liquid hot water, dilute acid, lime, and ammonia pretreatments (AFEX), could have potential as cost-effective pretreatments.

It should be noted that any such pretreatment procedure can be utilized to alter the biomass to make the mixture utilized in the invention. In this regard, the microorganism in step (i) can be adapted to apply all pretreatment procedures and their associated residual compound that can include, for example, furfural, hydroxymethyl furfural(HMF), phenolics like 3,4-dihydroxybenzal-dehyde, 3-methoxy-4-hydroxy-benzoic acid, cinnamic acid, anillin, vanillin alcohol, as well as sodium combinates like sodium hydroxide, nitrate combinates or ammonia, depending on the elected pretreatment method.

Acid pretreatment is a common pretreatment procedure. Acid pretreatment by acid hydrolysis and heat treatment can be utilized to produce the mixture inoculated in step (i) of the present invention. Any suitable acid can be used in this step, preferably an acid that hydrolyzes hemicelluloses away from cellulose. Some common acids that can be used include a mineral acid selected from hydrochloric acid, phosphoric acid, sulfuric acid, or sulfurous acid. Sulfuric acid, for example at concentration of about 0.5% to 2.0%, is preferred. Suitable organic acids may be carbonic acid, tartaric acid, citric acid, glucuronic acid, acetic acid, formic acid, or similar mono- or polycarboxylic acids. The acid pretreatment also typically involves heating the mixture, for example, in a range of about 70°C to 500°C, or in a range of about 120°C to 200°C.

Such acid pretreatment procedure can be used to generate the mixture utilized in step (i).

It should be noted that, when the biomass is obtained from plants, the mixture comprises at least one of cellulose, hemicellulose, lignin, furfural, phenolics and acetic acid.

In step (i), after the pretreatment procedure, the mixture is inoculated with at least one microorganism strain that is an extracellular cellulase producer. This microorganism can produce one or more cellulases that hydrolyze (enzymatic hydrolysis) at least one of cellulose and hemicelluloses present in the mixture under conditions to produce at least one of glucose, cellobiose, xylose, mannose, galactose, rhamnose, arabinose or other hemicellulose sugars.

Cellulase refers to a group of enzymes which hydrolyze cellulose, hemicellulose, and/or lignin. It is typically referred to as a class of enzymes produced by microorganisms (i.e., an extracellular cellulase producer), such as archaea, fungi, bacteria, protozoans, that catalyze the cellulolysis (or hydrolysis) of cellulose. However, it should be noted that there are cellulases produced by other kinds of microorganisms.

It is important to note that the present invention can utilize any extracellular and/or intracellular cellulase producer that produces one or more cellulases selected from the group consisting of: endoglucanase, exoglucanase, and β-glucosidase, hemicellulases, and laccase. Examples of cellulase producing microorganisms that can be utilized in the present invention include those in the attached Table 1.

Accordingly, the cellulase enzymes produced by the microorganism can perform enzymatic hydrolysis on the mixture in step (i). At the end of the enzymatic hydrolysis, the resultant medium can contains glucose, cellobiose, acetic acid, furfural, lignin, xylose, arabinose, mannose, galactose, and other hemicelluloses sugars.

Again, the present invention can utilize any microorganism that is an extracellular and/or intracellular cellulase enzyme producer to produce the requisite cellulase enzymes for enzymatic hydrolysis in step (i). As such, any prokaryote, including bacteria, archaea, and eukaryote, including fungi, which produces extracellular and/or intracellular cellulase enzymes may be utilized as the microorganism in step (i).

In one embodiment, the extracellular and/or intracellular cellulase producer is a fungus, archaea or bacteria of a genus selected from the group consisting of *Humicola, Trichoderma, Penicillium, Ruminococcus, Bacillus, Cytophaga* and *Sporocytophaga.* According to still a further embodiment the extracellular and/or intracellular cellulase producer can be at least microorganism selected from the group consisting of *Humicola grisea, Trichoderma harzianum, Trichoderma lignorum, Trichoderma reesei, Penicillium verruculosum, Ruminococcus albus, Bacillus subtilis, Bacillus thermoglucosidasius, Cytophaga spp.,* and *Sporocytophaga spp.*

In addition, a microorganism that is an extracellular and/or intracellular laccase enzyme producer may also be utilized in the present invention. Accordingly, any prokaryote, including bacteria, archaea, and eukaryote, including fungi, which produces extracellular and/or intracellular laccase may be utilized as the microorganism in step (i). In one embodiment, the extracellular and/or intracellular laccase producer is a fungus, bacteria or archaea of a genus selected from the group consisting of *Humicola, Trichoderma, Penicillium, Ruminococcus, Bacillus, Cytophaga* and *Sporocytophaga.* According to still a further embodiment the extracellular and/or intracellular laccase producer can be at least microorganism selected from the group consisting of *Humicola grisea, Trichoderma harzianum, Trichoderma lignorum, Trichoderma reesei, Penicillium verruculosum, Ruminococcus albus, Bacillus subtilis, Bacillus thermoglucosidasius, Cytophaga spp.,* and *Sporocytophaga spp.* Examples of laccase producing microorganisms that can be utilized in the present invention include those in the attached Table 1.

In one embodiment, the microorganism strain is a fungus, and more preferably, an aerobic fungus, such as *Trichoderma reesei.*

Again, any microorganism that is an extracellular and/or intracellular cellulase enzyme producer or extracellular and/or intracellular laccase enzyme producer can be utilized in the present invention to produce the requisite enzymes for enzymatic hydrolysis in step (i). Examples include those listed in attached Tables 1 and 2.

In the present invention, the type of microorganism can be selected and/or evolved to be specific to the type of plant biomass used.

The microorganism strain is tolerant to one or more compounds produced by the biomass pretreatment procedure, such as acid or alkaline pretreatment. Such compounds produced in the biomass pretreatment step include, for example, furfural, 3,4-dihydroxybenzaldehyde, 3-methoxy-4-hydroxy-benzoic acid, cinnamic acid, vanillin, vanillin alcohol, acetic acid, lignin and other residual salts or impurities.

In a preferred embodiment, the method of present invention utilizes at least one microorganism that has been evolutionarily modified and specialized for the specific type of biomass used. The evolutionarily modified microorganism can metabolize (enzymatic hydrolysis) the pretreated targeted biomass more efficiently and such microorganisms can be better able to tolerate residual compounds, for example, furfural and acetic acid. In this respect, the evolutionarily modified microorganism can have greater tolerance to furfural and acetic acid as compared to the unmodified wild-type version of the microorganism.

The evolutionarily modified microorganism can also produces one or more cellulase and/or laccase enzymes that are less inhibited by lignin and/or have improved capacity to metabolize lignin. As such, the evolutionarily modified microorganism can have improved capacity to produce enzymes (such as laccase) that metabolize lignin. Thus, the cellulase, hemicellulase and/or laccase enzymes produced by the evolutionarily modified microorganism can have greater capacity to metabolize cellulose and hemicelluloses with lignin as compared to the unmodified wild-type version of the microorganism.

Due to the use of the evolutionarily modified microorganism, the present invention allows for production of cellulases *in situ* in the mixture/medium of step (i). Consequently, there is no need to buy expensive cellulase enzymes from outside suppliers. This reduces operational costs as compared to conventional methods for biofuel production. Further, also due to the use of the evolutionarily modified microorganism, there is no need to wash and detoxify the acid pretreated mixture in the present invention to remove furfural, acetic acid, and salts that would normally inhibit biofuel production (as in conventional methods). By removing the wash and detoxification steps, the present invention can further reduces operational costs as compared to conventional methods for biofuel production.

It is noted that an evolutionarily modified microorganism is defined as a microorganism that has been modified by natural selection techniques. These techniques include, for example, serial transfer, serial dilution, Genetic Engine, continuous culture, and chemostat. One method and chemostatic device (the Genetic Engine; which can avoid dilution resistance in continuous culture) has been described in U.S. Patent No. 6, 686, 194-B1.

In one embodiment, the microorganism is evolutionarily modified by use of the continuous culture procedure as disclosed in PCT Application No. PCT/US05/05616, or United States Patent Application No. 11/508,286.

By cultivating a microorganism in this manner, beneficial mutations will occur to produce brand new alleles (i.e., variants of genes) that improve an organism's chances of survival and/or growth rate in that particular environment.

As such, the microorganism (e.g., fungi, archaea, algae, or bacteria) of the present invention can constitute a different strain, which can be identified by the mutations acquired during the course of culture, and these mutations, may allow the new cells to be distinguished from their ancestors' genotype characteristics. Thus, one can select new strains of microorganisms by segregating individuals with improved rates of reproduction through the process of natural selection.

Selection parameters for evolutionarily modifying the microorganism. By way of example, the microorganism in step (i) can be evolutionarily modified, through a natural selection technique, so that through evolution, it evolves to be adapted to use the particular carbon source selected. This involves identifying and selecting the fastest growing variant microorganisms, through adaptation in the natural selection technique utilized (such as continuous culture), that grow faster than wild-type on a particular carbon source. This also includes selecting those mutant microorganisms that have improved tolerance to furfural and acetic acid when using dilute acid pre-treatment; or selecting variant microorganisms that produce one or more cellulase and/or laccase enzymes that are less inhibited by lignin and/or have improved capacity to metabolize lignin. This would also involve selecting those microorganisms producing the above-discussed requisite cellulose enzymes.

It should be noted that, by using such parameters, any one of the natural selection techniques could be used in the present invention to evolutionarily modify the microorganism in the present invention.

Accordingly, the microorganisms can be evolutionarily modified in a number of ways so that their growth rate, viability, and utility as a biofuel, or other hydrocarbon product can be improved. Thus, the microorganisms can be evolutionarily modified to enhance their ability to grow on a particular substrate, constituted of the biomass and residual chemical related to chemical pre-treatment if any. In this regard, the microorganisms can be evolutionarily modified for a specific biomass plant and eventually associated residual chemicals.

The microorganisms (e.g., fungi, algae or bacteria) are preferably naturally occurring and have not been modified by recombinant DNA techniques. In other words, it is not necessary to genetically modify the microorganism to obtain a desired trait. Rather, the desired trait can be obtained by evolutionarily modifying the microorganism using the techniques discussed above. Nonetheless, even genetically modified microorganisms can be evolutionarily modified to increase their growth rate and/or viability of a modified by recombinant DNA techniques.

In one embodiment of the invention, the microorganism is a fungus, and in particular, *Trichoderma reesei* (**also known as *Hypocrea jecorina***), which has been evolutionarily modified by continuous culture.

The cellulase activity in step (i) can also be measured using common techniques to assess the level of cellulose activity to determine when to inhibit and/or stop the growth of the microorganism by proceeding to step (ii).

In step (ii) of the invention, growth and enzyme production of the microorganism is inhibited by one or more common techniques, such as those selected from the group consisting of: heat shock, UV exposure, radiation exposure, gas injection, and genetic modification of said at least one microorganism, (prior to step (i)) so that growth of said at least one genetically modified microorganism can be inhibited, for example, when temperature is increased to 45°C. Also, cells could be broken, using common techniques, for the release of intracellular cellulase enzymes in the supernatant.

Step (iii) of the invention involves inoculating the mixture of step (ii) with at least one algae strain that metabolizes said at least one of glucose, cellobiose, xylose or other hemicellulose sugars, under conditions so that said at least one algae strain produces one or more fatty acids.

Preferably, the growth of said at least one algae strain is not substantially inhibited by the presence of one or more of lignin, furfural, salts and cellulases enzymes present in the mixture.

The algae strain can also grow in one or more of the conditions selected from the group consisting of aerobic, anaerobic, phototrophic, and heterotrophic conditions.

Similar to the microorganism, the algae in step (iii) may be evolutionarily modified (using the natural selection techniques discussed above) to serve as an improved source of fatty acids, biofuel, biodiesel, and other hydrocarbon products. In this regard, the algae can be cultivated for use as a biofuel, biodiesel, or hydrocarbon based product.

Most algae need some amount of sunlight, carbon dioxide, and water. As a result, algae are often cultivated in open ponds and lakes. However, when algae are grown in such an "open" system, the systems are vulnerable to contamination by other algae and bacteria.

In one embodiment, the present invention can utilize heterotrophic algae (Stanier et al, Microbial World, Fifth Edition, Prentice-Hall, Englewood Cliffs, New Jersey, 1986, ), which can be grown in a closed reactor.

While a variety of algal species can be used, algae that naturally contain a high amount of lipids, for example, about 15-90%, about 30-80%, about 40-60%, or about 25-60% of lipids by dry weight of the algae is preferred. Prior to the work of the present invention, algae that naturally contained a high amount of lipids and high amount of bio-hydrocarbon were associated as having a slow growth rate. Evolutionarily modified algae strains can be produced in accordance with the present invention that exhibit an improved growth rate.

The conditions for growing the algae can be used to modify the algae. For example, there is considerable evidence that lipid accumulation takes place in algae as a response to the exhaustion of the nitrogen supply in the medium. Studies have analyzed samples where nitrogen has been removed from the culture medium and observed that while protein contents decrease under such conditions, the carbohydrate content increases, which are then followed by an increase in the lipid content of the algae. (Richardson et al, EFFECTS OF NITROGEN LIMITATION ON THE GROWTH OF ALGAE ON THE GROWTH AND COMPOSITION OF A UNICELLULAR ALGAE IN CONTINUOUS CULTURE CONDITIONS, Applied Microbiology, 1969, volume 18, page 2245-2250, 1969, ).

The algae can be evolutionarily modified by a number of techniques, including, for example, serial transfer, serial dilution, genetic engine, continuous culture, and chemostat. Any one of these techniques can be used to modify the algae. In one embodiment, the algae can be evolutionarily modified by continuous culture, as disclosed in PCT Application No. PCT/US05/05616, or United States Patent Application No. 11/508,286.

In doing so, the algae can be evolutionarily modified in a number of ways so that their growth rate, viability, and utility as a biofuel, or other hydrocarbon product can be improved. Accordingly, the algae can be evolutionarily modified to enhance their ability to grow on a particular substrate.

Selection parameters for evolutionarily modifying the algae. By way of example, the algae in step (iii) can be evolutionarily modified, through a natural selection technique, such as continuous culture, so that through evolution, the algae evolves to be adapted to use the particular carbon source selected. This involves identifying and selecting the fastest growing variant algae, through adaptation in the natural selection technique utilized, that grow faster than wild-type on a particular carbon source. This also includes, for example, selecting those algae that use acetic acid as a carbon source with improved tolerance to lignin, furfural and salts. It should be noted that, by using such parameters, any one of the natural selection techniques could be used in the present invention to evolutionarily modify the algae in the present invention.

In the present invention, such evolutionarily modified algae metabolize one or more compounds selected from the group consisting of: glucose, cellobiose, xylose, mannose, galactose, rhamnose, arabinose or other hemicellulose sugars and/or waste glycerol, and the algae use acetic acid a carbon source, under conditions so that said at least one algae strain produces one or more fatty acids. Such evolutionarily modified algae can also grow in one or more of the conditions selected from the group consisting of aerobic, anaerobic, phototrophic, and heterotrophic conditions.

In one embodiment, when step (iii) of the invention is performed under aerobic and heterotrophic conditions, the algae uses respiration.

In step (iii), the algae using the same amount of carbon source as an organism producing fermentation by-product producer, will produce only up to about 10% carbon dioxide. In this regard, more sugar is used by the algae for growth than is transformed to carbon dioxide. Alternatively, the microorganism or algae can be one that does not use fermentation, and as such much less carbon dioxide is made as a by-product in respiration.

Also, at least one algae strain in step (iii) preferably produces little or no inhibitory by-product, for growth inhibition of said algae.

Types of algae that can be utilized in the invention is one or more selected from the group consisting of green algae, red algae, blue-green algae, cyanobacteria and diatoms.

It should be noted that the present invention can utilize any algae strain that metabolizes at least one of glucose, cellobiose, xylose or other hemicellulose sugars, under conditions so that algae strain produces one or more fatty acids.

By way of example, the algae utilized in step (iii) can be from the following taxonomic divisions of algae:
(1) Division *Chlorophyta* (green algae);
(2) Division *Cyanophyta* (blue-green algae);
(3) Division *Bacillariophyta* (diatoms);
(4) Division *Chrysophyta;*
(5) Division *Xanthophyta;*
(6) Division *Cryptophyta;*
(7) Division *Euglenophyta;*
(8) Division *Ochrophyta ;*
(9) Division *Haptophyta;* and
(10) Division *Dinophyta.*

More specifically, the algae can be from the following species of algae, included within the above divisions (wherein number in parenthesis corresponds to the division):
Biddulphia (8);
*Pinguiococcus (8);*
*Skeletonema (8);*
*Emiliania (9);*
*Prymnesium (9);*
*Crypthecodinium (10);*
*Anabaenopsis circularis (2);*
*Ankistrodesmus braunii (1);*
*A. falcatus (1);*
*Botrydiopsis intercedens (5);*
*Bracteacoccus cinnabarinus (1);*
*B. engadiensis (1);*
*B. minor (Chodat) Petrova (1);*
*B. terrestris (1);*
*Bracteacoccus sp. (1);*
*Bracteacoccus sp. (1);*
*Bumilleriopsis brevis (5);*
*Chilomonas paramecium (6);*
*Chlamydobotrys sp. (1);*
*Chlamydomonas agloeformis (1);*
*C. dysosmos (1);*
*C. mundana Mojave strain Boron strain (1);*
*C. reinhardi* (-) *strain (1);*
*Chlorella ellipsoidea (1);*
*C. protothecoides (1);*
*C. pyrenoidosa (1);*
*C. pyrenoidosa ATCC 7516 (1);*
*C. pyrenoidosa C-37-2 (1);*
*C. pyrenoidosa Emerson (1);*
*C. pyrenoidosa 7-11-05 (1)*;
*C. vulgaris (1);*
*C. vulgaris ATCC 9765 (1);*
*C. vulgaris Emerson (1);*
*C. vulgaris Pratt-Trealease (1);*
*C. vulgaris var. viridis (1);*
*Chlorellidium tetrabotrys (5);*
*Chlorocloster engadinensis (5);*
*Chlorococcum macrostigmatum (1);*
*Chlorococcum sp. (1);*
*Chlorogloea fritschii (2);*
*Chlorogonium elongatum (1);*
*Coccomyxa elongata (1);*
*Cyclotella sp. (3);*
*Dictyochloris fragrans (1);*
*Euglena gracilis (7);*
*E. gracilis Vischer (7);*
*E. gracilis var. bacillaris (7);*
*E. gracilis var. saccharophila (7);*
*Haematococcus pluvialis (1);*
*Navicula incerta Grun. (3);*
*N. pelliculosa (3);*
*Neochloris alveolaris (1);*
*N. aquatica Starr (1);*
*N. gelatinosa Herndon (1);*
*N. pseudoalveolaris Deason (1);*
*Neochloris sp. (1);*
*Nitzschia angularis var. affinis (3) (Grun.) perag.;*
*N. chlosterium (Ehr.) (3);*
*N. curvilineata Hust. (3);*
*N. filiformis (3);*
*N. frustulum (Kurtz.) (3);*
*N. laevis Hust. (3);*
*Nostoc muscorum (2);*
*Ochromonas malhamensis (4);*
*Pediastrum boryanum (1);*
*P. duplex (1);*
*Polytoma obtusum (1);*
*P. ocellatum (1);*
*P. uvella (1);*
*Polytomella caeca (or coeca) (1);*
*Prototheca zopfii (1);*
*Scenedesmus acuminatus (1);*
*S. acutiformis (1);*
*S. costulatus Chod, var. chlorelloides (1);*
*S. dimorphus (1);*
*S. obliquus (1);*
*S. quadricauda (1);*
*Spongiochloris excentrica (1);*
*S. lamellata Deason (1);*
*S. spongiosus (1);*
*Spongiochloris sp. (1);*
*Spongiococcum alabamense (1);*
*S. excentricum (1);*
*S. excentricum Deason et Bold (1)*
*S. multinucleatum (1);*
*Stichococcus bacillaris (1);*
*S. subtilis (1);*
*Tolypothrix tenuis (2) ;*
*Tribonema aequale (5); and*
*T. minus (5).*

In one embodiment, the algae can be from *Chlorophyta (Chlorella and Prototheca), Prasinophyta (Dunaliella), Bacillariophyta (Navicula and Nitzschia), Ochrophyta (Ochromonas), Dinophyta (Gyrodinium) and Euglenozoa (Euglena).* More preferably, the algae is one selected from the group consisting of: *Monalanthus Salina; Botryococcus Braunii; Chlorella prototecoides; Outirococcus sp.; Scenedesmus obliquus; Nannochloris sp.; Dunaliella bardawil (D. Salina); Navicula pelliculosa; Radiosphaera negevensis; Biddulphia aurita; Chlorella vulgaris; Nitzschia palea; Ochromonas dannica; Chrorella pyrenoidosa; Peridinium cinctum; Neochloris oleabundans; Oocystis polymorpha; Chrysochromulina spp.; Scenedesmus acutus; Scenedesmus spp.; Chlorella minutissima; Prymnesium parvum; Navicula pelliculosa; Scenedesmus dimorphus; Scotiella sp.; Chorella spp.; Euglena gracilis;* and *Porphyridium cruentum.*

In another embodiment, the algae strain is *Chlorella protothecoides* and has been evolutionarily modified by continuous culture using the techniques and procedures described above.

Cyanobacteria may also be used with the present invention. Cyanobacteria are prokaryotes (single-celled organisms) often referred to as "blue-green algae." While most algae are eukaryotic, cyanobacteria are the most common exception. Cyanobacteria are generally unicellular, but can be found in colonial and filamentous forms, some of which differentiate into varying roles. For purposes of the claimed invention, cyanobacteria are considered algae.

*Chlorella protothecoides* and *Dunaliella Salina* are species that have been evolutionarily modified, cultivated, and harvested for production of a biodiesel.

The following publications relate to growing different types of algae and then harvesting algae for the purpose of producing biodiesel are incorporated herein by reference:
- Xu et al, HIGH QUALITY BIODESEL PRODUCTION FROM A MICROALGA CHLORELLA PROTHECOIDES BY HETEROTROPHIC GROWTH IN FERMENTERS, Journal of Biotechnology, vol. 126, 499-507, 2006,
- Kessler, Erich, PHYSIOLOGICAL AND BIOCHEMICAL CONTRIBUTIONS TO THE TAXONOMY OF THE GENUS PROTOTHECA, III. UTILIZATION OF ORGANIC CARBON AND NITROGEN COMPOUNDS, Arch Microbiol, volume 132, 103-106, 1982,
- Johnson D, 1987, OVERVIEW OF THE DOE/SERI AQUATIC SPECIES PROGRAM FY 1986 SOLAR ENERGY INSTITUTE,
- Pratt et al, PRODUCTION OF PROTEIN AND LIPID BY CHLORELLA VULGARIS AND CHLORELLA PYRENOIDOSA, Journal of Pharmaceutical Sciences, volume 52, Issue 10, 979-984 2006, and
- Sorokin, MAXIMUM GROWTH RATES OF CHLORELLA IN STEADY-STATE AND IN SYNCHRONIZED CULTURES, Proc. N.A.S, volume 45, 1740-1743, 1959.
- J.E. Zajic and Y.S. Chiu, HETEROTROPHIC CULTURE OF ALGAE, Biochemical Engineering, Faculty of Engineering Science, University of Western Ontario, London.

By employing the methods of the instant invention, the inoculation of the mixture with the at least one algae strain in step (iii) results in the algae metabolizing at least one of glucose, cellobiose, xylose, mannose, galactose, rhamnose, arabinose or other hemicellulose sugars, under conditions so that said at least one algae strain produces one or compounds, including fatty acids. In particular, the present invention in step (iii) involves culturing and growing the evolutionarily modified algae for extracellular and/or intracellular production of one or more compounds, such as fatty acids, hydrocarbons, proteins, pigments, sugars, such as polysaccharides and monosaccharides, and glycerol.

The resultant fatty acids, hydrocarbons, proteins, pigments, sugars, such as polysaccharides and monosaccharides, and glycerol in the algae can be used for biofuel, cosmetic, alimentary, mechanical grease, pigmentation, and medical use production.

In step (iv), the fatty acids, hydrocarbons, proteins, pigments, sugars, such as polysaccharides and monosaccharides, and glycerol can be recovered from the algae. The recovery step can be done by conventional techniques including one or more of fractionating the algae in the culture to obtain a fraction containing the compound, and other techniques including filtration-centrifugation, flocculation, solvent extraction, acid and base extraction, ultrasonication, microwave, pressing, distillation, thermal evaporation, homogenization, hydrocracking (fluid catalytic cracking), and drying of said at least one algae strain containing fatty acids.

In one embodiment, the resultant supernatant recovered in step (iv) can be reused.

Moreover, the recovered fatty acids can be optionally isolated and chemically treated (e.g., by transesterification), and thereby made into a biofuel (biodiesel) that can be incorporated into an engine fuel.

In this regard, the algae strain of the present invention produces hydrocarbon chains which can be used as feedstock for hydrocracking in an oil refinery to produce one or more compounds selected from the group consisting of octane, gasoline, petrol, kerosene, diesel and other petroleum product as solvent, plastic, oil, grease and fibers.

Direct transesterification can be performed on cells of the algae strain to produce fatty acids for biodiesel fuel. Methods of direct transesterification are well known and include breaking the algae cells, releasing fatty acids and transesterification through a base or acid method with methanol or ethanol to produce biodiesel fuel.

A further advantage of the method of the present invention is that the algae strain can be adapted to use waste glycerol, as a carbon source, produced by the transesterification reaction without pretreatment or refinement to produce fatty acids for biodiesel production.

Raw glycerol is the by-product of a transesterification reaction comprising glycerol and impurities such as fatty acid components, oily components, acid components, alkali components, soap components, alcohol component (e.g., methanol or ethanol) solvent (N-hexane) salts and/or diols. Due to the number and type of impurities present in raw glycerol, microorganisms exhibit little to no growth on the raw glycerol itself. However, the microorganism (e.g., algae or bacteria) can be evolutionarily modified to utilize raw glycerol as a primary carbon source.

The initial test for determining whether a particular type of microorganism will be able to grow in the presence of raw glycerol is the Refined Glycerol Test. The Refined Glycerol Test comprises culturing the microorganism in a medium comprising refined glycerol. The medium utilized in the Refined Glycerol Test may or may not have another carbon source such as glucose. However, the medium in the Refined Glycerol Test must contain a sufficient amount of glycerol so that it can be determined that the microorganism exhibits a minimum metabolizing capacity of the microorganism. The medium preferably contains 10ml-50 ml per liter of refined glycerol, 0.1ml-100ml per liter of refined glycerol, and 2ml-15ml per liter of refined glycerol.

If a positive result (i.e., the microorganism grows in the medium) is obtained with the Refined Glycerol Test, the microorganism can be evolutionarily modified to grow in a medium comprising raw glycerol. The culture medium preferably comprises, for example, 10-100% raw glycerol as a carbon source, 20-90% raw glycerol as a carbon source, 30-75% raw glycerol as a carbon source, 40-75% raw glycerol as a carbon source, or 50.01-55% raw glycerol as a carbon source. Indeed, some strains of microorganisms have been evolutionary modified to grow on a culture medium containing 100% raw glycerol.

An evolutionarily modified microorganism which produces extracellular and/or intracellular cellulase, hemicellulase, and laccase obtained in accordance with the present invention can have a maximum growth rate using the specific carbon sources in the pretreated biomass mixture of at least 5%, preferably 10%, 15%, 25%, 50%, 75%, 100%, 200%, 25%-100%, 25%-100%, 50%-150%, 25-200%, more than 200%, more than 300%, or more than 400% greater than microorganism of the same species that has not been evolutionarily modified to perform in the present invention.

An evolutionarily modified algae obtained in accordance with the present invention can have a maximum growth rate using, as a carbon source, the released polysaccharide and monosaccharide sugars from step (i) in the pretreated biomass mixture of at least 5%, preferably 10%, 15%, 25%, 50%, 75%, 100%, 200%, 25%-100%, 25%-100%, 50%-150%, 25-200%, more than 200%, more than 300%, or more than 400% greater than algae of the same species that has not been evolutionarily modified to perform in the present invention.

While it is envisioned that the most important commercial use for microorganisms grown from the by-products of biodiesel production will be to use the microorganisms themselves for products such as biofuel, biodiesel, "bio"-hydrocarbon products, renewable hydrocarbon products, and fatty acid based products, the invention is not limited to this embodiment. For example, if the microorganism is an algae, the algae could be grown from the by-products of biofuel production and harvested for use as a food, medicine, and nutritional supplement.

The biofuel obtained from the present invention may be used directly or as an alternative to petroleum for certain products.

In another embodiment, the biofuel (e.g., biodiesel) of the present invention may be used in a blend with other petroleum products or petroleum alternatives to obtain fuels such as motor gasoline and distillate fuel oil composition; finished nonfuel products such as solvents and lubricating oils; and feedstock for the petrochemical industry such as naphtha and various refinery gases.

For example, the biofuel as described above may be used directly in, or blended with other petroleum based compounds to produce solvents; paints; lacquers; and printing inks; lubricating oils; grease for automobile engines and other machinery; wax used in candy making, packaging, candles, matches, and polishes; petroleum jelly; asphalt; petroleum coke; and petroleum feedstock used as chemical feedstock derived from petroleum principally for the manufacture of chemicals, synthetic rubber, and a variety of plastics.

In a preferred embodiment, biodiesel produced in accordance with the present invention may be used in a diesel engine, or may be blended with petroleum-based distillate fuel oil composition at a ratio such that the resulting petroleum substitute may be in an amount of about 5-95%, 15-85%, 20-80%, 25-75%, 35-50% 50-75%, and 75-95% by weight of the total composition. The components may be mixed in any suitable manner.

The process of fueling a compression ignition internal combustion engine, comprises drawing air into a cylinder of a compression ignition internal combustion engine; compressing the air by a compression stroke of a piston in the cylinder; injecting into the compressed air, toward the end of the compression stroke, a fuel comprising the biodiesel; and igniting the fuel by heat of compression in the cylinder during operation of the compression ignition internal combustion engine.

In another embodiment, the biodiesel can be used as a lubricant or in a process of fueling a compression ignition internal combustion engine.

Alternatively, the biofuel may be further processed to obtain other hydrocarbons that are found in petroleum such as paraffins (e.g., methane, ethane, propane, butane, isobutane, pentane, and hexane), aromatics (e.g., benzene and naphthalene), cycloalkanes (e.g., cyclohexane and methyl cyclopentane), alkenes (e.g., ethylene, butene, and isobutene), alkynes (e.g., acetylene, and butadienes).

The resulting hydrocarbons can then in turn be used in petroleum based products such as solvents; paints; lacquers; and printing inks; lubricating oils; grease for automobile engines and other machinery; wax used in candy making, packaging, candles, matches, and polishes; petroleum jelly; asphalt; petroleum coke; and petroleum feedstock used as chemical feedstock derived from petroleum principally for the manufacture of chemicals, synthetic rubber, and a variety of plastics.

The following examples illustrate embodiments of the invention. It will be apparent that various changes and modifications can be made without departing from the scope of the invention as defined in the claims.

### Examples

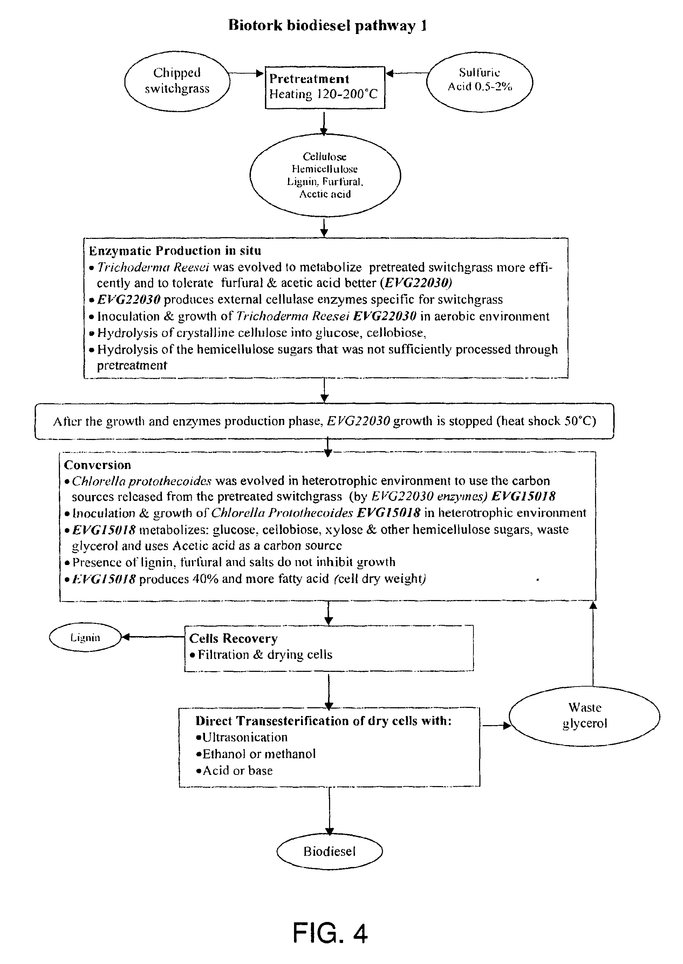

One exemplified embodiment of the method of the present invention can be found in the chart in Fig. 4 and is discussed below.

In this example, a plant biomass material of chipped switchgrass was subjected to pretreatment by acid hydrolysis (sulfuric acid 0.5% to 2.0%) and heat treatment (120°C -200°C).

This pretreatment procedure produced a mixture for use in the above-discussed step (i). This mixture contained cellulose, hemicellulose, lignin, furfural, and acetic acid.

In step (i), (Enzymatic Production *in situ)* the mixture was inoculated with an evolutionarily modified microorganism strain of *Trichoderma Reesei* having the following properties and under the following conditions:
- The modified *Trichoderma Reesei* strain was evolved to metabolize pretreated switchgrass more efficently and to tolerate furfural & acetic acid better (as was designated EVG22030).
- The strain produces external cellulase enzymes specific for switchgrass.
- Inoculation & growth of *Trichoderma Reesei* EVG22030 occurred in aerobic environment.
- Hydrolysis of crystalline cellulose into glucose, cellobiose.
- Hydrolysis of the hemicellulose sugars that was not sufficiently processed through pretreatment.

After the growth and enzymes production phase, *Trichoderma Reesei* EVG22030 growth is stopped by heat shock at 50°C (step (ii)).

In step (iii), the mixture from step (ii) was inoculated withan evolutionarily modified algae strain of *Chlorella protothecoides* having the following properties and under the following conditions:
- *Chlorella protothecoides* was evolved in heterotrophic environment to use the carbon sources released from the pretreated switchgrass (by EVG22030 enzymes) and designated EVG15018.
- Inoculation and growth of *Chlorella Protothecoides* EVG15018 in heterotrophic environment.
- EVG15018 metabolizes: glucose, cellobiose, xylose & other hemicellulose sugars, waste glycerol and uses acetic acid as a carbon source.
- Presence of lignin, furfural and salts do not inhibit growth.
- EVG15018 produces 40% and more fatty acid (cell dry weight).
- The algae were then grown under conditions and produced produces fatty acids.

The algae cells and fatty acids were then recovered by filtration and cell drying.

Direct transesterification was then performed on the dry cells (ultrasonication, through a base or acid method with methanol or ethanol) to produce biodiesel fuel. Waste glycerol was also recovered and recycled. The resultant biodiesel fuel can be directly used in any diesel engine for cars, trucks, generators, boats, etc.

While the invention has been described and pointed out in detail with reference to operative embodiments thereof it will be understood by those skilled in the art that various changes, modifications, substitutions and omissions can be made without departing from the spirit of the invention. It is intended, that the invention is defined by the claims which follow.

**TABLE 1 - EXAMPLES OF MICRO-ORGANISMS PRODUCING EXTRA- AND/OR INTRA-CELLULAR CELLULASE ENZYMES**

| | Division | Organism |
|---|---|---|
| Archaea | Crenarchaeota | Caldivirga maquilingensis |
| Archaea | Crenarchaeota | Sulfolobus acidocaldarius |
| Archaea | Crenarchaeota | Sulfolobus solfataricus |
| Archaea | Crenarchaeota | Thermofilum pendens |
| Archaea | Euryarchaeota | Picrophilus torridus |
| Archaea | Euryarchaeota | Pyrococcus abyssi |
| Archaea | Euryarchaeota | Pyrococcus furiosus |
| Archaea | Euryarchaeota | Pyrococcus horikoshii |
| Archaea | Euryarchaeota | Thermoplasma volcanium |
| Bacteria | Acidobacteria | Acidobacterium capsulatum |
| Bacteria | Actinobacteria | Acidothermus cellulolyticus |
| Bacteria | Actinobacteria | Actinomadura sp. |
| Bacteria | Actinobacteria | Actinomyces sp. |
| Bacteria | Actinobacteria | Amycolatopsis orientalis |
| Bacteria | Actinobacteria | Arthrobacter aurescens |
| Bacteria | Actinobacteria | Arthrobacter sp. |
| Bacteria | Actinobacteria | Bifidobacterium adolescentis |
| Bacteria | Actinobacteria | Bifidobacterium animalis |
| Bacteria | Actinobacteria | Bifidobacterium bifidum |
| Bacteria | Actinobacteria | Bifidobacterium longum |
| Bacteria | Actinobacteria | Cellulomonas fimi |
| Bacteria | Actinobacteria | Cellulomonas flavigena |
| Bacteria | Actinobacteria | Cellulomonas pachnodae |
| Bacteria | Actinobacteria | Cellulomonas uda |
| Bacteria | Actinobacteria | Cellulosimicrobium sp. |
| Bacteria | Actinobacteria | Clavibacter michiganensis subsp. michiganensis |
| Bacteria | Actinobacteria | Clavibacter michiganensis subsp. sepedonicus |
| Bacteria | Actinobacteria | Frankia alni |
| Bacteria | Actinobacteria | Frankia sp. |
| Bacteria | Actinobacteria | Jonesia sp. |
| Bacteria | Actinobacteria | Kineococcus radiotolerans |
| Bacteria | Actinobacteria | Leifsonia xyli subsp. xyli |
| Bacteria | Actinobacteria | Microbispora bispora |
| Bacteria | Actinobacteria | Micromonospora cellulolyticum |
| Bacteria | Actinobacteria | Mycobacterium abscessus |
| Bacteria | Actinobacteria | Mycobacterium avium |
| Bacteria | Actinobacteria | Mycobacterium avium subsp. Paratuberculosis |
| Bacteria | Actinobacteria | Mycobacterium bovis |
| Bacteria | Actinobacteria | Mycobacterium gilvum |
| Bacteria | Actinobacteria | Mycobacterium marinum |
| Bacteria | Actinobacteria | Mycobacterium smegmatis |
| Bacteria | Actinobacteria | Mycobacterium sp. |
| Bacteria | Actinobacteria | Mycobacterium tuberculosis |
| Bacteria | Actinobacteria | Mycobacterium ulcerans |
| Bacteria | Actinobacteria | Mycobacterium vanbaalenii |
| Bacteria | Actinobacteria | Mycobacterium vanbaalenii |
| Bacteria | Actinobacteria | Nocardioides sp. |
| Bacteria | Actinobacteria | Propionibacterium acnes |
| Bacteria | Actinobacteria | Rhodococcus equi |
| Bacteria | Actinobacteria | Saccharopolyspora erythraea |
| Bacteria | Actinobacteria | Saccharothrix australiensis |
| Bacteria | Actinobacteria | Salinispora arenicola |
| Bacteria | Actinobacteria | Salinispora tropica |
| Bacteria | Actinobacteria | Streptomyces ambofaciens |
| Bacteria | Actinobacteria | Streptomyces avermitilis |
| Bacteria | Actinobacteria | Streptomyces chartreusis |
| Bacteria | Actinobacteria | Streptomyces chattanoogensis |
| Bacteria | Actinobacteria | Streptomyces coelicolor |
| Bacteria | Actinobacteria | Streptomyces fradiae var. |
| Bacteria | Actinobacteria | Streptomyces griseus |
| Bacteria | Actinobacteria | Streptomyces griseus subsp. griseus |
| Bacteria | Actinobacteria | Streptomyces halstedii |
| Bacteria | Actinobacteria | Streptomyces lividans |
| Bacteria | Actinobacteria | Streptomyces nanchangensis |
| Bacteria | Actinobacteria | Streptomyces olivaceoviridis |
| Bacteria | Actinobacteria | Streptomyces reticuli |
| Bacteria | Actinobacteria | Streptomyces roseiscleroticus |
| Bacteria | Actinobacteria | Streptomyces sp. |
| Bacteria | Actinobacteria | Streptomyces thermocyaneoviolaceus |
| Bacteria | Actinobacteria | Streptomyces thermoviolaceus |
| Bacteria | Actinobacteria | Streptomyces turgidiscabies |
| Bacteria | Actinobacteria | Streptomyces viridosporus |
| Bacteria | Actinobacteria | Thermobifida alba |
| Bacteria | Actinobacteria | Thermobifida fusca |
| Bacteria | Actinobacteria | Thermopolyspora flexuosa |
| Bacteria | Bacteroidetes | Bacteroides cellulosolvens |
| Bacteria | Bacteroidetes | Bacteroides fragilis |
| Bacteria | Bacteroidetes | Bacteroides ovatus |
| Bacteria | Bacteroidetes | Bacteroides thetaiotaomicron |
| Bacteria | Bacteroidetes | Bacteroides vulgatus |
| Bacteria | Bacteroidetes | Cytophaga hutchinsonii |
| Bacteria | Bacteroidetes | Cytophaga xylanolytica |
| | Division | Organism |
| Bacteria | Bacteroidetes | Flavobacterium johnsoniae |
| Bacteria | Bacteroidetes | Flavobacterium psychrophilum |
| Bacteria | Bacteroidetes | Flavobacterium sp. |
| Bacteria | Bacteroidetes | Gramella forsetii |
| Bacteria | Bacteroidetes | Parabacteroides distasonis |
| Bacteria | Bacteroidetes | Prevotella bryantii |
| Bacteria | Bacteroidetes | Prevotella ruminicola |
| Bacteria | Bacteroidetes | Rhodothermus marinus |
| Bacteria | Chlorobi | Chlorobium chlorochromatii |
| Bacteria | Chlorobi | Pelodictyon luteolum |
| Bacteria | Chloroflexi | Chloroflexus aurantiacus |
| Bacteria | Chloroflexi | Herpetosiphon aurantiacus |
| Bacteria | Chloroflexi | Roseiflexus castenholzii |
| Bacteria | Chloroflexi | Roseiflexus sp. |
| Bacteria | Cyanobacteria | Anabaena variabilis |
| Bacteria | Cyanobacteria | Nostoc punctiforme |
| Bacteria | Cyanobacteria | Nostoc sp. |
| Bacteria | Cyanobacteria | Synechococcus elongatus |
| Bacteria | Cyanobacteria | Synechococcus sp. |
| Bacteria | Cyanobacteria | Synechocystis sp. |
| Bacteria | Deinococcus-Thermus | Deinococcus geothermalis |
| Bacteria | Deinococcus-Thermus | Thermus caldophilus |
| Bacteria | Dictyoglomi | Dictyoglomus thermophilum |
| Bacteria | Fibrobacteres | Fibrobacter intestinalis |
| Bacteria | Fibrobacteres | Fibrobacter succinogenes |
| Bacteria | Fibrobacteres | Fibrobacter succinogenes subsp. succinogenes |
| Bacteria | Firmicutes | Acetivibrio cellulolyticus |
| Bacteria | Firmicutes | Alicyclobacillus acidocaldarius |
| Bacteria | Firmicutes | Alkaliphilus metalliredigens |
| Bacteria | Firmicutes | Anoxybacillus kestanbolensis |
| Bacteria | Firmicutes | Bacillus agaradhaerens |
| Bacteria | Firmicutes | Bacillus alcalophilus |
| Bacteria | Firmicutes | Bacillus amyloliquefaciens |
| Bacteria | Firmicutes | Bacillus anthracis |
| Bacteria | Firmicutes | Bacillus cereus |
| Bacteria | Firmicutes | Bacillus circulans |
| Bacteria | Firmicutes | Bacillus clausii |
| Bacteria | Firmicutes | Bacillus firmus |
| Bacteria | Firmicutes | Bacillus halodurans |
| Bacteria | Firmicutes | Bacillus licheniformis |
| Bacteria | Firmicutes | Bacillus plakortiensis |
| Bacteria | Firmicutes | Bacillus pumilus |
| | Division | Organism |
| Bacteria | Firmicutes | Bacillus sp. |
| Bacteria | Firmicutes | Bacillus subtilis |
| Bacteria | Firmicutes | Bacillus subtilis subsp. subtilis |
| Bacteria | Firmicutes | Bacillus thuringiensis serovar alesti |
| Bacteria | Firmicutes | Bacillus thuringiensis serovar canadensis |
| Bacteria | Firmicutes | Bacillus thuringiensis serovar darmstadiensis |
| Bacteria | Firmicutes | Bacillus thuringiensis serovar israelensis |
| Bacteria | Firmicutes | Bacillus thuringiensis serovar morrisoni |
| Bacteria | Firmicutes | Bacillus thuringiensis serovar san diego |
| Bacteria | Firmicutes | Bacillus thuringiensis serovar sotto |
| Bacteria | Firmicutes | Bacillus thuringiensis serovar thompsoni |
| Bacteria | Firmicutes | Bacillus thuringiensis serovar tochigiensis |
| Bacteria | Firmicutes | Butyrivibrio fibrisolvens |
| Bacteria | Firmicutes | Caldicellulosiruptor saccharolyticus |
| Bacteria | Firmicutes | Caldicellulosiruptor sp. |
| Bacteria | Firmicutes | Clostridium acetobutylicum |
| Bacteria | Firmicutes | Clostridium beijerinckii |
| Bacteria | Firmicutes | Clostridium cellulolyticum |
| Bacteria | Firmicutes | Clostridium cellulovorans |
| Bacteria | Firmicutes | Clostridium difficile |
| Bacteria | Firmicutes | Clostridium josui |
| Bacteria | Firmicutes | Clostridium lentocellum |
| Bacteria | Firmicutes | Clostridium longisporum |
| Bacteria | Firmicutes | Clostridium phytofermentans |
| Bacteria | Firmicutes | Clostridium phytofermentans |
| Bacteria | Firmicutes | Clostridium saccharobutylicum |
| Bacteria | Firmicutes | Clostridium sp. |
| Bacteria | Firmicutes | Clostridium stercorarium |
| Bacteria | Firmicutes | Clostridium thermocellum |
| Bacteria | Firmicutes | Eubacterium cellulosolvens |
| Bacteria | Firmicutes | Eubacterium ruminantium |
| Bacteria | Firmicutes | Geobacillus caldoxylosilyticus |
| Bacteria | Firmicutes | Geobacillus stearothermophilus |
| Bacteria | Firmicutes | Geobacillus thermodenitrificans |
| Bacteria | Firmicutes | Geobacillus thermoleovorans |
| Bacteria | Firmicutes | Lactobacillus acidophilus |
| Bacteria | Firmicutes | Lactobacillus brevis |
| Bacteria | Firmicutes | Lactobacillus gasseri |
| Bacteria | Firmicutes | Lactobacillus johnsonii |
| | Division | Organism |
| Bacteria | Firmicutes | Lactobacillus reuteri |
| Bacteria | Firmicutes | Lactococcus lactis subsp. cremoris |
| Bacteria | Firmicutes | Lactococcus lactis subsp. lactis |
| Bacteria | Firmicutes | Leuconostoc mesenteroides subsp. Mesenteroides |
| Bacteria | Firmicutes | Listeria innocua |
| Bacteria | Firmicutes | Listeria monocytogenes |
| Bacteria | Firmicutes | Paenibacillus barcinonensis |
| Bacteria | Firmicutes | Paenibacillus curdlanolyticus |
| Bacteria | Firmicutes | Paenibacillus fukuinensis |
| Bacteria | Firmicutes | Paenibacillus lautus |
| Bacteria | Firmicutes | Paenibacillus pabuli |
| Bacteria | Firmicutes | Paenibacillus polymyxa |
| Bacteria | Firmicutes | Paenibacillus sp. |
| Bacteria | Firmicutes | Ruminococcus albus |
| Bacteria | Firmicutes | Ruminococcus flavefaciens |
| Bacteria | Firmicutes | Streptococcus mutans |
| Bacteria | Firmicutes | Streptococcus sanguinis |
| Bacteria | Firmicutes | Syntrophomonas wolfei subsp. wolfei |
| Bacteria | Firmicutes | Thermoanaerobacter pseudethanolicus |
| Bacteria | Firmicutes | Thermoanaerobacter sp. |
| Bacteria | Firmicutes | Thermoanaerobacter tengcongensis |
| Bacteria | Firmicutes | Thermoanaerobacterium polysaccharolyticum |
| Bacteria | Firmicutes | Thermoanaerobacterium saccharolyticum |
| Bacteria | Firmicutes | Thermoanaerobacterium sp. |
| Bacteria | Firmicutes | Thermoanaerobacterium thermosulfurigenes |
| Bacteria | Firmicutes | Thermobacillus xylanilyticus |
| Bacteria | Fusobacteria | Fusobacterium mortiferum |
| Bacteria | Planctomycetes | Rhodopirellula baltica |
| Bacteria | Proteobacteria | Acidiphilium cryptum |
| Bacteria | Proteobacteria | Acidovorax avenae subsp. citrulli |
| Bacteria | Proteobacteria | Acinetobacter baumannii |
| Bacteria | Proteobacteria | Aeromonas hydrophila |
| Bacteria | Proteobacteria | Aeromonas hydrophila subsp. hydrophila |
| Bacteria | Proteobacteria | Aeromonas punctata |
| Bacteria | Proteobacteria | Aeromonas salmonicida subsp. salmonicida |
| Bacteria | Proteobacteria | Agrobacterium tumefaciens |
| Bacteria | Proteobacteria | Alcaligenes sp. |
| Bacteria | Proteobacteria | Anaeromyxobacter dehalogenans |
| Bacteria | Proteobacteria | Anaeromyxobacter sp. |
| Bacteria | Proteobacteria | Asaia bogorensis |
| | Division | Organism |
| Bacteria | Proteobacteria | Azoarcus sp. |
| Bacteria | Proteobacteria | Azorhizobium caulinodans |
| Bacteria | Proteobacteria | Beijerinckia indica subsp. indica |
| Bacteria | Proteobacteria | Bordetella avium |
| Bacteria | Proteobacteria | Bradyrhizobium japonicum |
| Bacteria | Proteobacteria | Brucella abortus |
| Bacteria | Proteobacteria | Brucella canis |
| Bacteria | Proteobacteria | Brucella melitensis |
| Bacteria | Proteobacteria | Brucella ovis |
| Bacteria | Proteobacteria | Brucella suis |
| Bacteria | Proteobacteria | Burkholderia ambifaria |
| Bacteria | Proteobacteria | Burkholderia ambifaria |
| Bacteria | Proteobacteria | Burkholderia cenocepacia |
| Bacteria | Proteobacteria | Burkholderia cepacia |
| Bacteria | Proteobacteria | Burkholderia mallei |
| Bacteria | Proteobacteria | Burkholderia multivorans |
| Bacteria | Proteobacteria | Burkholderia phymatum |
| Bacteria | Proteobacteria | Burkholderia phytofirmans |
| Bacteria | Proteobacteria | Burkholderia pseudomallei |
| Bacteria | Proteobacteria | Burkholderia sp. |
| Bacteria | Proteobacteria | Burkholderia sp. |
| Bacteria | Proteobacteria | Burkholderia thailandensis |
| Bacteria | Proteobacteria | Burkholderia vietnamiensis |
| Bacteria | Proteobacteria | Burkholderia xenovorans |
| Bacteria | Proteobacteria | Caulobacter crescentus |
| Bacteria | Proteobacteria | Caulobacter sp. |
| Bacteria | Proteobacteria | Cellvibrio japonicus (formerly Pseudomonas cellulosa) |
| Bacteria | Proteobacteria | Cellvibrio mixtus |
| Bacteria | Proteobacteria | Chromobacterium violaceum |
| Bacteria | Proteobacteria | Citrobacter koseri |
| Bacteria | Proteobacteria | Colwellia psychrerythraea |
| Bacteria | Proteobacteria | Enterobacter cloacae |
| Bacteria | Proteobacteria | Enterobacter cloacae |
| Bacteria | Proteobacteria | Enterobacter sakazakii |
| Bacteria | Proteobacteria | Enterobacter sp. |
| Bacteria | Proteobacteria | Erwinia carotovora |
| Bacteria | Proteobacteria | Erwinia carotovora subsp. Atroseptica |
| Bacteria | Proteobacteria | Erwinia chrysanthemi |
| Bacteria | Proteobacteria | Erwinia rhapontici |
| Bacteria | Proteobacteria | Erwinia tasmaniensis |
| Bacteria | Proteobacteria | Escherichia coli |
| Bacteria | Proteobacteria | Gluconacetobacter diazotrophicus |
| Bacteria | Proteobacteria | Gluconacetobacter xylinus |
| Bacteria | Proteobacteria | Hahella chejuensis |
| | Division | Organism |
| Bacteria | Proteobacteria | Halorhodospira halophila |
| Bacteria | Proteobacteria | Klebsiella pneumoniae |
| Bacteria | Proteobacteria | Klebsiella pneumoniae subsp. pneumoniae |
| Bacteria | Proteobacteria | Legionella pneumophila Lens |
| Bacteria | Proteobacteria | Legionella pneumophila Paris |
| Bacteria | Proteobacteria | Legionella pneumophila str. Corby |
| Bacteria | Proteobacteria | Legionella pneumophila subsp. Pneumophila |
| Bacteria | Proteobacteria | Leptothrix cholodnii |
| Bacteria | Proteobacteria | Leptothrix cholodnii |
| Bacteria | Proteobacteria | Lysobacter sp. |
| Bacteria | Proteobacteria | Maricaulis maris |
| Bacteria | Proteobacteria | Marinomonas sp. |
| Bacteria | Proteobacteria | Mesorhizobium loti |
| Bacteria | Proteobacteria | Methylobacillus flagellatus |
| Bacteria | Proteobacteria | Methylobacterium extorquens |
| Bacteria | Proteobacteria | Methylobacterium radiotolerans |
| Bacteria | Proteobacteria | Methylobacterium sp. |
| Bacteria | Proteobacteria | Myxococcus xanthus |
| Bacteria | Proteobacteria | Nitrosospira multiformis |
| Bacteria | Proteobacteria | Parvibaculum lavamentivorans |
| Bacteria | Proteobacteria | Pectobacterium carotovorum |
| Bacteria | Proteobacteria | Pectobacterium carotovorum atroseptica |
| Bacteria | Proteobacteria | Pectobacterium carotovorum subsp. carotovorum |
| Bacteria | Proteobacteria | Photobacterium profundum |
| Bacteria | Proteobacteria | Polaromonas sp. |
| Bacteria | Proteobacteria | Polynucleobacter sp. |
| Bacteria | Proteobacteria | Proteus mirabilis |
| Bacteria | Proteobacteria | Pseudoalteromonas atlantica |
| Bacteria | Proteobacteria | Pseudoalteromonas atlantica |
| Bacteria | Proteobacteria | Pseudoalteromonas haloplanktis |
| Bacteria | Proteobacteria | Pseudoalteromonas sp. |
| Bacteria | Proteobacteria | Pseudomonas entomophila |
| Bacteria | Proteobacteria | Pseudomonas fluorescens |
| Bacteria | Proteobacteria | Pseudomonas putida |
| Bacteria | Proteobacteria | Pseudomonas sp. |
| Bacteria | Proteobacteria | Pseudomonas stutzeri |
| Bacteria | Proteobacteria | Pseudomonas syringae pv. mori |
| Bacteria | Proteobacteria | Pseudomonas syringae pv. phaseolicola |
| Bacteria | Proteobacteria | Pseudomonas syringae pv. syringae |
| Bacteria | Proteobacteria | Pseudomonas syringae pv. Tomato |
| Bacteria | Proteobacteria | Psychromonas ingrahamii |
| | Division | Organism |
| Bacteria | Proteobacteria | Ralstonia eutropha |
| Bacteria | Proteobacteria | Ralstonia metallidurans |
| Bacteria | Proteobacteria | Ralstonia solanacearum |
| Bacteria | Proteobacteria | Ralstonia syzygii |
| Bacteria | Proteobacteria | Rhizobium etli |
| Bacteria | Proteobacteria | Rhizobium leguminosarum bv. trifolii |
| Bacteria | Proteobacteria | Rhizobium sp. |
| Bacteria | Proteobacteria | Rhodobacter sphaeroides |
| Bacteria | Proteobacteria | Rhodoferax ferrireducens |
| Bacteria | Proteobacteria | Rhodopseudomonas palustris |
| Bacteria | Proteobacteria | Saccharophagus degradans |
| Bacteria | Proteobacteria | Salmonella enterica subsp. arizonae |
| Bacteria | Proteobacteria | Salmonella typhimurium |
| Bacteria | Proteobacteria | Serratia proteamaculans |
| Bacteria | Proteobacteria | Shigella boydii |
| Bacteria | Proteobacteria | Shigella flexneri |
| Bacteria | Proteobacteria | Shigella sonnei |
| Bacteria | Proteobacteria | Sinorhizobium medicae |
| Bacteria | Proteobacteria | Sinorhizobium meliloti |
| Bacteria | Proteobacteria | Sorangium cellulosum |
| Bacteria | Proteobacteria | Stigmatella aurantiaca |
| Bacteria | Proteobacteria | Teredinibacter turnerae |
| Bacteria | Proteobacteria | Thiobacillus denitrificans |
| Bacteria | Proteobacteria | Vibrio cholerae |
| Bacteria | Proteobacteria | Vibrio fischeri |
| Bacteria | Proteobacteria | Vibrio harveyi |
| Bacteria | Proteobacteria | Vibrio parahaemolyticus |
| Bacteria | Proteobacteria | Vibrio sp. |
| Bacteria | Proteobacteria | Vibrio vulnificus |
| Bacteria | Proteobacteria | Xanthomonas albilineans |
| Bacteria | Proteobacteria | Xanthomonas axonopodis pv. citri str. |
| Bacteria | Proteobacteria | Xanthomonas campestris pv. campestris |
| Bacteria | Proteobacteria | Xanthomonas campestris pv. vesicatoria |
| Bacteria | Proteobacteria | Xanthomonas oryzae pv. oryzae |
| Bacteria | Proteobacteria | Xylella fastidiosa |
| Bacteria | Proteobacteria | Yersinia enterocolitica subsp. enterocolitica |
| Bacteria | Proteobacteria | Yersinia enterocolitica subsp. enterocolitica |
| Bacteria | Proteobacteria | Yersinia pestis |
| Bacteria | Proteobacteria | Yersinia pestis |
| Bacteria | Proteobacteria | Yersinia pestis Antiqua |
| Bacteria | Proteobacteria | Yersinia pestis biovar Medievalis |
| Bacteria | Proteobacteria | Yersinia pseudotuberculosis |
| | Division | Organism |
| Bacteria | Proteobacteria | Yersinia pseudotuberculosis |
| Bacteria | Proteobacteria | Zymomonas mobilis subsp. mobilis |
| Bacteria | Spirochaetes | Leptospira biflexa |
| Bacteria | Spirochaetes | Leptospira borgpetersenii |
| Bacteria | Spirochaetes | Leptospira interrogans |
| Bacteria | Thermotogae | Fervidobacterium nodosum |
| Bacteria | Thermotogae | Petrotoga mobilis |
| Bacteria | Thermotogae | Thermotoga lettingae |
| Bacteria | Thermotogae | Thermotoga maritima |
| Bacteria | Thermotogae | Thermotoga neapolitana |
| Bacteria | Thermotogae | Thermotoga petrophila |
| Bacteria | Thermotogae | Thermotoga sp. |
| Bacteria | Verrucomicrobia | Opitutus terrae |
| Eukaryota | Ascomycota | Acremonium cellulolyticus |
| Eukaryota | Ascomycota | Acremonium sp. |
| Eukaryota | Ascomycota | Acremonium thermophilum |
| Eukaryota | Ascomycota | Alternaria alternata |
| Eukaryota | Ascomycota | Aspergillus aculeatus |
| Eukaryota | Ascomycota | Aspergillus flavus |
| Eukaryota | Ascomycota | Aspergillus fumigatus |
| Eukaryota | Ascomycota | Aspergillus kawachii |
| Eukaryota | Ascomycota | Aspergillus nidulans |
| Eukaryota | Ascomycota | Aspergillus niger |
| Eukaryota | Ascomycota | Aspergillus oryzae |
| Eukaryota | Ascomycota | Aspergillus sojae |
| Eukaryota | Ascomycota | Aspergillus sp. |
| Eukaryota | Ascomycota | Aspergillus sulphureus |
| Eukaryota | Ascomycota | Aspergillus terreus |
| Eukaryota | Ascomycota | Aspergillus tubingensis |
| Eukaryota | Ascomycota | Aspergillus versicolor |
| Eukaryota | Ascomycota | Aureobasidium pullulans var. melanigenum |
| Eukaryota | Ascomycota | Beltraniella portoricensis |
| Eukaryota | Ascomycota | Bionectria ochroleuca |
| Eukaryota | Ascomycota | Blumeria graminis |
| Eukaryota | Ascomycota | Botryosphaeria rhodina |
| Eukaryota | Ascomycota | Botryotinia fuckeliana |
| Eukaryota | Ascomycota | Candida albicans |
| Eukaryota | Ascomycota | Candida glabrata |
| Eukaryota | Ascomycota | Candida oleophila |
| Eukaryota | Ascomycota | Chaetomidium pingtungium |
| Eukaryota | Ascomycota | Chaetomium brasiliense |
| Eukaryota | Ascomycota | Chaetomium thermophilum |
| Eukaryota | Ascomycota | Chaetomium thermophilum var. thermophilum |
| | Division | Organism |
| Eukaryota | Ascomycota | Chrysosporium lucknowense |
| Eukaryota | Ascomycota | Claviceps purpurea |
| Eukaryota | Ascomycota | Coccidioides posadasii |
| Eukaryota | Ascomycota | Cochliobolus heterostrophus |
| Eukaryota | Ascomycota | Coniothyrium minitans |
| Eukaryota | Ascomycota | Corynascus heterothallicus |
| Eukaryota | Ascomycota | Cryphonectria parasitica |
| Eukaryota | Ascomycota | Cryptovalsa sp. |
| Eukaryota | Ascomycota | Cylindrocarpon sp. |
| Eukaryota | Ascomycota | Daldinia eschscholzii |
| Eukaryota | Ascomycota | Debaryomyces hansenii |
| Eukaryota | Ascomycota | Debaryomyces occidentalis |
| Eukaryota | Ascomycota | Emericella desertorum |
| Eukaryota | Ascomycota | Emericella nidulans |
| Eukaryota | Ascomycota | Epichloe festucae |
| Eukaryota | Ascomycota | Eremothecium gossypii |
| Eukaryota | Ascomycota | Fusarium anguioides |
| Eukaryota | Ascomycota | Fusarium chlamydosporum |
| Eukaryota | Ascomycota | Fusarium culmorum |
| Eukaryota | Ascomycota | Fusarium equiseti |
| Eukaryota | Ascomycota | Fusarium lateritium |
| Eukaryota | Ascomycota | Fusarium oxysporum |
| Eukaryota | Ascomycota | Fusarium poae |
| Eukaryota | Ascomycota | Fusarium proliferatum |
| Eukaryota | Ascomycota | Fusarium sp. |
| Eukaryota | Ascomycota | Fusarium tricinctum |
| Eukaryota | Ascomycota | Fusarium udum |
| Eukaryota | Ascomycota | Fusarium venenatum |
| Eukaryota | Ascomycota | Fusicoccum sp. |
| Eukaryota | Ascomycota | Geotrichum sp. |
| Eukaryota | Ascomycota | Gibberella avenacea |
| Eukaryota | Ascomycota | Gibberella moniliformis |
| Eukaryota | Ascomycota | Gibberella pulicaris |
| Eukaryota | Ascomycota | Gibberella zeae |
| Eukaryota | Ascomycota | Gliocladium catenulatum |
| Eukaryota | Ascomycota | Humicola grisea |
| Eukaryota | Ascomycota | Humicola grisea var. thermoidea |
| Eukaryota | Ascomycota | Humicola insolens |
| Eukaryota | Ascomycota | Humicola nigrescens |
| Eukaryota | Ascomycota | Hypocrea jecorina |
| Eukaryota | Ascomycota | Hypocrea koningii |
| Eukaryota | Ascomycota | Hypocrea lixii |
| Eukaryota | Ascomycota | Hypocrea pseudokoningii |
| Eukaryota | Ascomycota | Hypocrea schweinitzii |
| Eukaryota | Ascomycota | Hypocrea virens |
| | Division | Organism |
| Eukaryota | Ascomycota | Kluyveromyces lactis |
| Eukaryota | Ascomycota | Lacazia loboi |
| Eukaryota | Ascomycota | Leptosphaeria maculans |
| Eukaryota | Ascomycota | Macrophomina phaseolina |
| Eukaryota | Ascomycota | Magnaporthe grisea |
| Eukaryota | Ascomycota | Malbranchea cinnamomea |
| Eukaryota | Ascomycota | Melanocarpus |
| Eukaryota | Ascomycota | Melanocarpus albomyces |
| Eukaryota | Ascomycota | Nectria haematococca |
| Eukaryota | Ascomycota | Nectria ipomoeae |
| Eukaryota | Ascomycota | Neotyphodium lolii |
| Eukaryota | Ascomycota | Neotyphodium sp. |
| Eukaryota | Ascomycota | Neurospora crassa |
| Eukaryota | Ascomycota | Nigrospora sp. |
| Eukaryota | Ascomycota | Paecilomyces lilacinus |
| Eukaryota | Ascomycota | Paracoccidioides brasiliensis (various strains) |
| Eukaryota | Ascomycota | Penicillium canescens |
| Eukaryota | Ascomycota | Penicillium chrysogenum |
| Eukaryota | Ascomycota | Penicillium citrinum |
| Eukaryota | Ascomycota | Penicillium decumbens |
| Eukaryota | Ascomycota | Penicillium funiculosum |
| Eukaryota | Ascomycota | Penicillium janthinellum |
| Eukaryota | Ascomycota | Penicillium occitanis |
| Eukaryota | Ascomycota | Penicillium oxalicum |
| Eukaryota | Ascomycota | Penicillium purpurogenum |
| Eukaryota | Ascomycota | Penicillium simplicissimum |
| Eukaryota | Ascomycota | Pichia angusta |
| Eukaryota | Ascomycota | Pichia anomala |
| Eukaryota | Ascomycota | Pichia guilliermondii |
| Eukaryota | Ascomycota | Pichia pastoris |
| Eukaryota | Ascomycota | Pichia stipitis |
| Eukaryota | Ascomycota | Pseudoplectania nigrella |
| Eukaryota | Ascomycota | Robillarda sp. |
| Eukaryota | Ascomycota | Saccharomyces bayanus |
| Eukaryota | Ascomycota | Saccharomyces castellii |
| Eukaryota | Ascomycota | Saccharomyces cerevisiae |
| Eukaryota | Ascomycota | Saccharomyces kluyveri |
| Eukaryota | Ascomycota | Saccobolus dilutellus |
| Eukaryota | Ascomycota | Sarcoscypha occidentalis |
| Eukaryota | Ascomycota | Schizosaccharomyces pombe |
| Eukaryota | Ascomycota | Scopulariopsis brevicaulis |
| Eukaryota | Ascomycota | Scytalidium thermophilum |
| Eukaryota | Ascomycota | Stachybotrys chartarum |
| Eukaryota | Ascomycota | Stachybotrys echinata |
| | Division | Organism |
| Eukaryota | Ascomycota | Staphylotrichum coccosporum |
| Eukaryota | Ascomycota | Stilbella annulata |
| Eukaryota | Ascomycota | Talaromyces emersonii |
| Eukaryota | Ascomycota | Thermoascus aurantiacus |
| Eukaryota | Ascomycota | Thermoascus aurantiacus var. levisporus |
| Eukaryota | Ascomycota | Thermomyces lanuginosus |
| Eukaryota | Ascomycota | Thermomyces verrucosus |
| Eukaryota | Ascomycota | Thielavia australiensis |
| Eukaryota | Ascomycota | Thielavia microspora |
| Eukaryota | Ascomycota | Thielavia terrestris |
| Eukaryota | Ascomycota | Trichoderma asperellum |
| Eukaryota | Ascomycota | Trichoderma longibrachiatum |
| Eukaryota | Ascomycota | Trichoderma parceramosum |
| Eukaryota | Ascomycota | Trichoderma sp. |
| Eukaryota | Ascomycota | Trichoderma viride |
| Eukaryota | Ascomycota | Trichophaea saccata |
| Eukaryota | Ascomycota | Trichothecium roseum |
| Eukaryota | Ascomycota | Verticillium dahliae |
| Eukaryota | Ascomycota | Verticillium fungicola |
| Eukaryota | Ascomycota | Verticillium tenerum |
| Eukaryota | Ascomycota | Volutella colletotrichoides |
| Eukaryota | Ascomycota | Xylaria polymorpha |
| Eukaryota | Ascomycota | Yarrowia lipolytica |
| Eukaryota | Basidiomycota | Agaricus bisporus |
| Eukaryota | Basidiomycota | Armillariella tabescens |
| Eukaryota | Basidiomycota | Athelia rolfsii |
| Eukaryota | Basidiomycota | Chlorophyllum molybdites |
| Eukaryota | Basidiomycota | Clitocybe nuda |
| Eukaryota | Basidiomycota | Clitopilus prunulus |
| Eukaryota | Basidiomycota | Coprinopsis cinerea |
| Eukaryota | Basidiomycota | Crinipellis stipitaria |
| Eukaryota | Basidiomycota | Cryptococcus adeliensis |
| Eukaryota | Basidiomycota | Cryptococcus flavus |
| Eukaryota | Basidiomycota | Cryptococcus neoformans |
| Eukaryota | Basidiomycota | Cryptococcus neoformans var. neoformans |
| | Division | Organism |
| Eukaryota | Basidiomycota | Cryptococcus sp. |
| Eukaryota | Basidiomycota | Exidia glandulosa |
| Eukaryota | Basidiomycota | Filobasidium floriforme (Cryptococcus albidus) |
| Eukaryota | Basidiomycota | Fomitopsis palustris |
| Eukaryota | Basidiomycota | Gloeophyllum sepiarium |
| Eukaryota | Basidiomycota | Gloeophyllum trabeum |
| | Division | Organism |
| Eukaryota | Basidiomycota | Infundibulicybe gibba |
| Eukaryota | Basidiomycota | Irpex lacteus |
| Eukaryota | Basidiomycota | Lentinula edodes |
| Eukaryota | Basidiomycota | Meripilus giganteus |
| Eukaryota | Basidiomycota | Phanerochaete chrysosporium |
| Eukaryota | Basidiomycota | Pleurotus sajor-caju |
| Eukaryota | Basidiomycota | Pleurotus sp. |
| Eukaryota | Basidiomycota | Polyporus arcularius |
| Eukaryota | Basidiomycota | Schizophyllum commune |
| Eukaryota | Basidiomycota | Trametes hirsuta |
| Eukaryota | Basidiomycota | Trametes versicolor |
| Eukaryota | Basidiomycota | Ustilago maydis |
| Eukaryota | Basidiomycota | Volvariella volvacea |
| Eukaryota | Basidiomycota | Xylaria hypoxylon |
| Eukaryota | Chlorophyta | Chlorella vulgaris |
| Eukaryota | Chytridiomycota | Anaeromyces sp. |
| Eukaryota | Chytridiomycota | Neocallimastix frontalis |
| Eukaryota | Chytridiomycota | Neocallimastix patriciarum |
| Eukaryota | Chytridiomycota | Neocallimastix sp. |
| Eukaryota | Chytridiomycota | Orpinomyces joyonii |
| Eukaryota | Chytridiomycota | Orpinomyces sp. |
| Eukaryota | Cnidaria | Hydra magnipapillata |
| Eukaryota | Mycetozoa | Dictyostelium discoideum |
| Eukaryota | Ochrophyta | Eisenia andrei |
| Eukaryota | Oomycota | Phytophthora cinnamomi |
| Eukaryota | Oomycota | Phytophthora infestans |
| Eukaryota | Oomycota | Phytophthora ramorum |
| Eukaryota | Oomycota | Phytophthora sojae |
| Eukaryota | Prasinophyta | Ostreococcus lucimarinus |
| Eukaryota | Prasinophyta | Ostreococcus tauri |
| Eukaryota | Zygomycota | Mucor circinelloides |
| Eukaryota | Zygomycota | Phycomyces nitens |
| Eukaryota | Zygomycota | Poitrasia circinans |
| Eukaryota | Zygomycota | Rhizopus oryzae |
| Eukaryota | Zygomycota | Syncephalastrum racemosum |

**TABLES 2 - EXAMPLES OF MICRO-ORGANISMS PRODUCING EXTRA- AND/OR INTRA-CELLULAR LACCASE ENZYMES**

| | Division | Organism |
|---|---|---|
| Eukaryota | Ascomycota | Alternaria alternata |
| Eukaryota | Ascomycota | Arxula adeninivorans |
| Eukaryota | Ascomycota | Ashbya gossypii |
| Eukaryota | Ascomycota | Aspergillus fumigatus |
| Eukaryota | Ascomycota | Aspergillus niger |
| Eukaryota | Ascomycota | Aspergillus oryzae |
| Eukaryota | Ascomycota | Aspergillus terreus |
| Eukaryota | Ascomycota | Botryotinia fuckeliana |
| Eukaryota | Ascomycota | Buergenerula spartinae |
| Eukaryota | Ascomycota | Candida albicans |
| Eukaryota | Ascomycota | Candida glabrata |
| Eukaryota | Ascomycota | Chaetomium globosum |
| Eukaryota | Ascomycota | Chaetomium thermophilum var. thermophilum |
| Eukaryota | Ascomycota | Claviceps purpurea |
| Eukaryota | Ascomycota | Coccidioides immitis |
| Eukaryota | Ascomycota | Colletotrichum lagenarium |
| Eukaryota | Ascomycota | Corynascus heterothallicus |
| Eukaryota | Ascomycota | Cryphonectria parasitica |
| Eukaryota | Ascomycota | Cryptococcus bacillisporus |
| Eukaryota | Ascomycota | Cryptococcus gattii |
| Eukaryota | Ascomycota | Cryptococcus neoformans |
| Eukaryota | Ascomycota | Cryptococcus neoformans var. neoformans |
| Eukaryota | Ascomycota | Davidiella tassiana |
| Eukaryota | Ascomycota | Debaryomyces hansenii |
| Eukaryota | Ascomycota | Emericella nidulans |
| Eukaryota | Ascomycota | Fusarium oxysporum |
| Eukaryota | Ascomycota | Fusarium oxysporum f. sp. lycopersici |
| Eukaryota | Ascomycota | Fusarium proliferatum |
| Eukaryota | Ascomycota | Gaeumannomyces graminis |
| Eukaryota | Ascomycota | Gaeumannomyces graminis var. graminis |
| Eukaryota | Ascomycota | Gaeumannomyces graminis var. tritici |
| Eukaryota | Ascomycota | Gibberella zeae |
| Eukaryota | Ascomycota | Glomerella cingulata |
| Eukaryota | Ascomycota | Hortaea acidophila |
| Eukaryota | Ascomycota | Humicola insolens |
| | Division | Organism |
| Eukaryota | Ascomycota | Hypomyces rosellus |
| Eukaryota | Ascomycota | Hypoxylon sp. |
| Eukaryota | Ascomycota | Kluyveromyces lactis |
| Eukaryota | Ascomycota | Lachnum spartinae |
| Eukaryota | Ascomycota | Lactarius blennius |
| Eukaryota | Ascomycota | Lactarius subdulcis |
| Eukaryota | Ascomycota | Melanocarpus albomyces |
| Eukaryota | Ascomycota | Morchella conica |
| Eukaryota | Ascomycota | Morchella crassipes |
| Eukaryota | Ascomycota | Morchella elata |
| Eukaryota | Ascomycota | Morchella esculenta |
| Eukaryota | Ascomycota | Morchella sp. |
| Eukaryota | Ascomycota | Morchella spongiola |
| Eukaryota | Ascomycota | Mycosphaerella sp. |
| Eukaryota | Ascomycota | Neurospora crassa |
| Eukaryota | Ascomycota | Paracoccidioides brasiliensis |
| Eukaryota | Ascomycota | Penicillium adametzii |
| Eukaryota | Ascomycota | Penicillium amagasakiense |
| Eukaryota | Ascomycota | Penicillium expansum |
| Eukaryota | Ascomycota | Penicillium simplissimum |
| Eukaryota | Ascomycota | Penicillium variabile |
| Eukaryota | Ascomycota | Phaeosphaeria halima |
| Eukaryota | Ascomycota | Phaeosphaeria spartinicola |
| Eukaryota | Ascomycota | Pichia pastoris |
| Eukaryota | Ascomycota | Pleospora spartinae |
| Eukaryota | Ascomycota | Podospora anserina |
| Eukaryota | Ascomycota | Saccharomyces cerevisiae |
| Eukaryota | Ascomycota | Saccharomyces pastorianus |
| Eukaryota | Ascomycota | Schizosaccharomyces pombe |
| Eukaryota | Ascomycota | Stagonospora sp. |
| Eukaryota | Ascomycota | Talaromyces flavus |
| Eukaryota | Ascomycota | Verpa conica |
| Eukaryota | Ascomycota | Yarrowia lipolytica |
| Eukaryota | Basidiomycota | Agaricus bisporus |
| Eukaryota | Basidiomycota | Amanita citrina |
| Eukaryota | Basidiomycota | Amylostereum areolatum |
| Eukaryota | Basidiomycota | Amylostereum chailletii |
| Eukaryota | Basidiomycota | Amylostereum ferreum |
| Eukaryota | Basidiomycota | Amylostereum laevigatum |
| Eukaryota | Basidiomycota | Amylostereum sp. |
| Eukaryota | Basidiomycota | Athelia rolfsii |
| Eukaryota | Basidiomycota | Auricularia auricula-judae |
| Eukaryota | Basidiomycota | Auricularia polytricha |
| | Division | Organism |
| Eukaryota | Basidiomycota | Bjerkandera adusta |
| Eukaryota | Basidiomycota | Bjerkandera sp. |
| Eukaryota | Basidiomycota | Bondarzewia montana |
| Eukaryota | Basidiomycota | Ceriporiopsis rivulosa |
| Eukaryota | Basidiomycota | Ceriporiopsis subvermispora |
| Eukaryota | Basidiomycota | Cerrena unicolor |
| Eukaryota | Basidiomycota | Climacocystis borealis |
| Eukaryota | Basidiomycota | Clitocybe nebularis |
| Eukaryota | Basidiomycota | Clitocybe quercina |
| Eukaryota | Basidiomycota | Collybia butyracea |
| Eukaryota | Basidiomycota | Coniophora puteana |
| Eukaryota | Basidiomycota | Coprinellus congregatus |
| Eukaryota | Basidiomycota | Coprinellus disseminatus |
| Eukaryota | Basidiomycota | Coprinopsis cinerea |
| Eukaryota | Basidiomycota | Coprinopsis cinerea okayama |
| Eukaryota | Basidiomycota | Coriolopsis gallica |
| Eukaryota | Basidiomycota | Cortinarius flexipes |
| Eukaryota | Basidiomycota | Crinipellis sp. |
| Eukaryota | Basidiomycota | Cyathus bulleri |
| Eukaryota | Basidiomycota | Cyathus sp. |
| Eukaryota | Basidiomycota | Daedalea quercina |
| Eukaryota | Basidiomycota | Dichomitus squalens |
| Eukaryota | Basidiomycota | Echinodontium japonicum |
| Eukaryota | Basidiomycota | Echinodontium tinctorium |
| Eukaryota | Basidiomycota | Echinodontium tsugicola |
| Eukaryota | Basidiomycota | Filobasidiella neoformans |
| Eukaryota | Basidiomycota | Flammulina velutipes |
| Eukaryota | Basidiomycota | Funalia trogii |
| Eukaryota | Basidiomycota | Ganoderma applanatum |
| Eukaryota | Basidiomycota | Ganoderma australe |
| Eukaryota | Basidiomycota | Ganoderma formosanum |
| Eukaryota | Basidiomycota | Ganoderma lucidum |
| Eukaryota | Basidiomycota | Ganoderma sp. |
| Eukaryota | Basidiomycota | Ganoderma tsunodae |
| Eukaryota | Basidiomycota | Gloeophyllum trabeum |
| Eukaryota | Basidiomycota | Grifola frondosa |
| Eukaryota | Basidiomycota | Gymnopus fusipes |
| Eukaryota | Basidiomycota | Gymnopus peronatus |
| Eukaryota | Basidiomycota | Gyromitra esculenta |
| Eukaryota | Basidiomycota | Halocyphina villosa |
| Eukaryota | Basidiomycota | Hebeloma radicosum |
| Eukaryota | Basidiomycota | Heterobasidion abietinum |
| Eukaryota | Basidiomycota | Heterobasidion annosum |
| Eukaryota | Basidiomycota | Heterobasidion araucariae |
| | Division | Organism |
| Eukaryota | Basidiomycota | Heterobasidion insulare |
| Eukaryota | Basidiomycota | Heterobasidion parviporum |
| Eukaryota | Basidiomycota | Hypholoma sp. |
| Eukaryota | Basidiomycota | Irpex lacteus |
| Eukaryota | Basidiomycota | Lentinula edodes |
| Eukaryota | Basidiomycota | Lentinus tigrinus |
| Eukaryota | Basidiomycota | Lepista flaccida |
| Eukaryota | Basidiomycota | Lepista irina |
| Eukaryota | Basidiomycota | Lepista nuda |
| Eukaryota | Basidiomycota | Lyophyllum shimeji |
| Eukaryota | Basidiomycota | Macrolepiota procera |
| Eukaryota | Basidiomycota | Macrotyphula juncea |
| Eukaryota | Basidiomycota | Malassezia sympodialis |
| Eukaryota | Basidiomycota | Marasmius alliaceus |
| Eukaryota | Basidiomycota | Megacollybia platyphylla |
| Eukaryota | Basidiomycota | Mycena cinerella |
| Eukaryota | Basidiomycota | Mycena crocata |
| Eukaryota | Basidiomycota | Mycena galopus |
| Eukaryota | Basidiomycota | Mycena rosea |
| Eukaryota | Basidiomycota | Mycena zephirus |
| Eukaryota | Basidiomycota | Panus rudis |
| Eukaryota | Basidiomycota | Panus sp. |
| Eukaryota | Basidiomycota | Paxillus involutus |
| Eukaryota | Basidiomycota | Peniophora sp. |
| Eukaryota | Basidiomycota | Phanerochaete chrysosporium |
| Eukaryota | Basidiomycota | Phanerochaete flavidoalba |
| Eukaryota | Basidiomycota | Phanerochaete sordida |
| Eukaryota | Basidiomycota | Phlebia radiata |
| Eukaryota | Basidiomycota | Phlebiopsis gigantea |
| Eukaryota | Basidiomycota | Piloderma byssinum |
| Eukaryota | Basidiomycota | Piriformospora indica |
| Eukaryota | Basidiomycota | Pleurotus cornucopiae |
| Eukaryota | Basidiomycota | Pleurotus eryngii |
| Eukaryota | Basidiomycota | Pleurotus ostreatus |
| Eukaryota | Basidiomycota | Pleurotus pulmonarius |
| Eukaryota | Basidiomycota | Pleurotus sajor-caju |
| Eukaryota | Basidiomycota | Pleurotus sapidus |
| Eukaryota | Basidiomycota | Pleurotus sp. 'Florida' |
| Eukaryota | Basidiomycota | Polyporus alveolaris |
| Eukaryota | Basidiomycota | Polyporus ciliatus |
| Eukaryota | Basidiomycota | Psathyrella corrugis |
| Eukaryota | Basidiomycota | Psathyrella dicrani |
| Eukaryota | Basidiomycota | Psathyrella murcida |
| Eukaryota | Basidiomycota | Pycnoporus cinnabarinus |
| Eukaryota | Basidiomycota | Pycnoporus coccineus |
| | Division | Organism |
| Eukaryota | Basidiomycota | Pycnoporus sanguineus |
| Eukaryota | Basidiomycota | Rigidoporus microporus |
| Eukaryota | Basidiomycota | Russula atropurpurea |
| Eukaryota | Basidiomycota | Russula mairei |
| Eukaryota | Basidiomycota | Russula nigricans |
| Eukaryota | Basidiomycota | Russula ochroleuca |
| Eukaryota | Basidiomycota | Schizopora paradoxa |
| Eukaryota | Basidiomycota | Schizophyllum commune |
| Eukaryota | Basidiomycota | Schizophyllum commune f. trop. radiatum |
| Eukaryota | Basidiomycota | Spongipellis sp. |
| Eukaryota | Basidiomycota | Stropharia squamosa |
| Eukaryota | Basidiomycota | Termitomyces sp. |
| Eukaryota | Basidiomycota | Thanatephorus cucumeris |
| Eukaryota | Basidiomycota | Trametes cervina |
| Eukaryota | Basidiomycota | Trametes hirsuta |
| Eukaryota | Basidiomycota | Trametes ochracea |
| Eukaryota | Basidiomycota | Trametes pubescens |
| Eukaryota | Basidiomycota | Trametes sp. |
| Eukaryota | Basidiomycota | Trametes versicolor |
| Eukaryota | Basidiomycota | Trametes villosa |
| Eukaryota | Basidiomycota | Ustilago maydis |
| Eukaryota | Basidiomycota | Volvariella volvacea |
| Eukaryota | Basidiomycota | Xerocomus chrysenteron |
| Eukaryota | Basidiomycota | Xylaria sp. |

## Claims

1. A method of producing fatty acids, the method comprising:
(i) inoculating a mixture of at least one of cellulose, hemicellulose, and lignin with at least one microorganism strain that produces one or more cellulase, hemicellulase and laccase, that hydrolyze at least one of cellulose, hemicellulose and lignin, under conditions to produce at least one of glucose, cellobiose, xylose, mannose, galactose, rhamnose, arabinose or other hemicellulose sugars;
(ii) inhibiting growth of said at least one microorganism strain; and
(iii) inoculating the mixture of step (ii) with at least one algae strain that metabolizes said at least one of glucose, cellobiose, xylose, mannose, galactose, rhamnose, arabinose or other hemicellulose sugars, under conditions so that said at least one algae strain produces one or more fatty acids.

2. The method of claim 1, wherein the mixture in step (i) further comprises at least one of furfural, phenolics compounds and acetic acid.

3. The method of claim 1, wherein the mixture in step (i) is obtained from a biomass.

4. The method of claim 3, wherein said biomass comprises plant biomass.

5. The method of claim 4, wherein said biomass is obtained from plant or animal waste.

6. The method of claim 4, wherein said plant biomass undergoes pretreatment by acid hydrolysis and heat treatment to produce said mixture inoculated in step (i).

7. The method of claim 4, wherein said plant biomass comprises:
10-35% lignin;
15-35% hemicellulose; and
30-60% cellulose.

8. The method of claim 1, wherein said at least one microorganism strain is an extracellular and/or intracellular cellulase, hemicellulase and laccase enzyme producer microorganism.

9. The method of claim 8, wherein said extracellular and/or intracellular cellulase producer microorganism is selected from the group consisting of: prokaryote, bacteria, archaea, and eukaryote, and fungi.

10. The method of claim 1, wherein said at least one microorganism strain is tolerant to one or more compounds produced by a pretreatment of the biomass, wherein said one or more compounds are selected from the group consisting of: furfural, acetic acid, and other impurities.

11. The method of claim 1, wherein said one or more cellulases is at least one selected from the group consisting of: endoglucanase, exoglucanase, and β-glucosidase, and hemicellulases and optionally laccase.

12. The method of claim 1, further comprising measuring cellulase and/or hemicellulase activity in step (i), and depending on the activity of the enzyme, proceeding to step (ii).

13. The method of claim 1, wherein said at least one algae strain in step (iii) metabolizes said at least one of glucose, cellobiose, xylose, mannose, galactose, rhamnose, arabinose or other hemicellulose sugars, and waste glycerol.

14. The method of claim 1, wherein said at least one algae strain in step (iii) uses acetic acid as a carbon source.

15. The method of claim 1, wherein when step (iii) is under aerobic and heterotrophic conditions, said at least one algae strain uses respiration.

16. The method of claim 1, further comprising (iv) recovering said one or more fatty acids from said at least one algae strain.

17. The method of claim 1, wherein step (iii) further comprises culturing and growing said at least one algae strain under conditions for extracellular and/or intracellular production of at least one compound selected from the group consisting of fatty acids, hydrocarbons, proteins, pigments, sugars, such as polysaccharides and monosaccharides, and glycerol.

18. The method of claim 16, further comprising, after step (iv), direct transesterification of cells of said at least one algae strain to produce fatty acids for biodiesel fuel.

19. The method of claim 18, wherein the direct transesterification comprises breaking the algae cells, releasing fatty acids and transesterification through a base or acid method with methanol or ethanol to produce biodiesel fuel.

20. The method of claim 1, wherein said at least one algae strain is adapted to use waste glycerol, as carbon source, produced by the transesterification reaction without pretreatment or refinement to produce fatty acids for biodiesel production.

## Patentansprüche

1. Verfahren zur Herstellung von Fettsäuren, umfassend:
(i) Animpfen einer Mischung von zumindest einem Mitglied aus der Gruppe aus Cellulose, Hemicellulose und Lignin mit zumindest einem Mikroorganismenstamm, der eine oder mehr Cellulase(n), Hemicellulase(n) und Laccase(n) produziert, die zumindest ein Mitglied aus der Gruppe aus Cellulose, Hemicellulose und Lignin hydrolysieren, unter Bedingungen, um zumindest eine Verbindung aus der Gruppe aus Glukose, Cellobiose, Xylose, Mannose, Galaktose, Rhamnose, Arabinose oder anderen Hemicellulosezuckern zu produzieren;
(ii) Inhibieren des Wachstums des zumindest einen Mikroorganismenstamms; und
(iii) Animpfen der Mischung aus Schritt (ii) mit zumindest einem Algenstamm, der die zumindest eine Verbindung aus der Gruppe aus Glukose, Cellobiose, Xylose, Mannose, Galaktose, Rhamnose, Arabinose oder anderen Hemicellulosezuckern metabolisiert, unter Bedingungen, so daß der zumindest eine Algenstamm eine oder mehrere Fettsäure(n) produziert.

2. Verfahren nach Anspruch 1, worin die Mischung in Schritt (i) weiter zumindest eine Verbindung aus Furfural, phenolischen Verbindungen und Essigsäure umfaßt.

3. Verfahren nach Anspruch 1, worin die Mischung in Schritt (i) aus Biomasse erhalten wird.

4. Verfahren nach Anspruch 3, worin die Biomasse Pflanzenbiomasse umfaßt.

5. Verfahren nach Anspruch 4, worin die Biomasse aus Pflanzen- oder Tierabfällen erhalten wird.

6. Verfahren nach Anspruch 4, worin die Pflanzenbiomasse einer Vorbehandlung durch saure Hydrolyse und Wärmebehandlung unterzogen wird, um die in Schritt (i) angeimpfte Mischung herzustellen.

7. Verfahren nach Anspruch 4, worin die Pflanzenbiomasse umfaßt:
10-35% Lignin
15-35% Hemicellulose; und
30-60% Cellulose.

8. Verfahren nach Anspruch 1, worin der zumindest eine Mikroorganismenstamm ein extrazellulärer und/oder intrazellulärer Cellulase-, Hemicellulase- und Laccase-Enzym produzierender Mikroorganismus ist.

9. Verfahren nach Anspruch 8, worin der extrazelluläre und/oder intrazelluläre Cellulase produzierende Mikroorganismus ausgewählt ist aus der Gruppe bestehend aus: Prokaryoten, Bakterien, Archaea, Eukaryoten und Pilzen.

10. Verfahren nach Anspruch 1, worin der zumindest eine Mikroorganismenstamm tolerant gegenüber einer oder mehreren Verbindungen ist, die durch eine Vorbehandlung der Biomasse produziert wurden, worin die eine oder mehreren Verbindungen gewählt sind aus der Gruppe bestehend aus: Fufural, Essigsäure und anderen Verunreinigungen.

11. Verfahren nach Anspruch 1, worin die eine oder mehreren Cellulase(n) zumindest eine ist, gewählt aus der Gruppe bestehend aus: Endoglucanase, Exoglucanase und β-Glucosidase und Hemicellulasen und optional Laccase.

12. Verfahren nach Anspruch 1, weiter umfassend das Messen der Cellulase- und/oder Hemicellulaseaktivität in Schritt (i) und abhängig von der Aktivität des Enzyms, fortfahren mit Schritt (ii).

13. Verfahren nach Anspruch 1, worin der zumindest eine Algenstamm in Schritt (iii) zumindest eine aus Glukose, Cellobiose, Xylose, Mannose, Galaktose, Rhamnose, Arabinose oder anderen Hemicellulosezuckern und Abfall-Glycerol metabolisiert.

14. Verfahren nach Anspruch 1, worin der zumindest eine Algenstamm in Schritt (iii) Essigsäure als Kohlenstoffquelle nutzt.

15. Verfahren nach Anspruch 1, worin, wenn Schritt (iii) unter Aeroben und heterotrophen Bedingungen stattfindet, der zumindest eine Algenstamm die Atmung nutzt.

16. Verfahren nach Anspruch 1, weiter umfassend (iv) Rückgewinnung der einen oder mehreren Fettsäuren aus dem zumindest einen Algenstamm.

17. Verfahren nach Anspruch 1, worin Schritt (iii) weiter das Kultivieren und Wachsen des zumindest einen Algenstamms umfaßt, unter Bedingungen für die extrazelluläre und/oder intrazelluläre Produktion von zumindest einer Verbindung, ausgewählt aus der Gruppe bestehend aus Fettsäuren, Kohlenwasserstoffen, Proteinen, Pigmenten, Zuckern, wie z.B. Polysacchariden und Monosacchariden, und Glycerol.

18. Verfahren nach Anspruch 16, weiter umfassend nach Schritt (iv) die unmittelbare Umesterung von Zellen des zumindest einen Algenstamms, um Fettsäuren für Biodieselkraftstoff herzustellen.

19. Verfahren nach Anspruch 18, worin die direkte Umesterung das Aufbrechen der Algenzellen umfaßt, wodurch Fettsäuren freigesetzt werden, und die Umesterung durch ein basisches oder saures Verfahren mit Methanol oder Ethanol, um Biodieselkraftstoff herzustellen.

20. Verfahren nach Anspruch 1, worin der zumindest eine Algenstamm angepaßt ist, um Abfall-Glycerol als Kohlenstoffquelle zu nutzen, hergestellt durch die Umesterungsreaktion ohne Vorbehandlung oder Veredelung, um Fettsäuren für die Biodieselherstellung zu produzieren.

## Revendications

1. Procédé de production d'acides gras, le procédé comprenant :
(i) l'inoculation d'un mélange d'au moins un élément parmi la cellulose, l'hémicellulose et la lignine avec au moins une souche de microorganisme qui produit un ou plusieurs élément(s) parmi la cellulase, l'hémicellulase et la laccase, qui hydrolysent au moins un élément parmi la cellulose, l'hémicellulose et la lignine, dans des conditions permettant de produire au moins un élément parmi le glucose, le cellobiose, le xylose, le mannose, le galactose, le rhamnose, l'arabinose ou d'autres sucres d'hémicellulose ;
(ii) l'inhibition de la croissance de ladite au moins une souche de microorganisme ; et
(iii) l'inoculation du mélange de l'étape (ii) avec au moins une souche d'algue qui métabolise ledit au moins un élément parmi le glucose, le cellobiose, le xylose, le mannose, le galactose, le rhamnose, l'arabinose ou autres sucres d'hémicellulose dans des conditions telles que ladite au moins une souche d'algue produit un ou plusieurs acide(s) gras.

2. Procédé selon la revendication 1, dans lequel le mélange de l'étape (i) comprend en outre au moins un élément parmi le furfural, des composés phénoliques et de l'acide acétique.

3. Procédé selon la revendication 1, dans lequel le mélange de l'étape (i) est obtenu à partir d'une biomasse.

4. Procédé selon la revendication 3, dans lequel ladite biomasse comprend une biomasse végétale.

5. Procédé selon la revendication 4, dans lequel ladite biomasse est obtenue à partir de déchets végétaux ou animaux.

6. Procédé selon la revendication 4, dans lequel ladite biomasse végétale subit un prétraitement par hydrolyse acide et un traitement thermique pour produire ledit mélange inoculé à l'étape (i).

7. Procédé selon la revendication 4, dans lequel ladite biomasse végétale comprend :
de 10 à 35 % de lignine ;
de 15 à 35 % d'hémicellulose ; et
de 30 à 60 % de cellulose.

8. Procédé selon la revendication 1, dans lequel ladite au moins une souche de microorganisme est un microorganisme producteur d'enzyme de cellulase, d'hémicellulase et de laccase extracellulaire et/ou intracellulaire.

9. Procédé selon la revendication 8, dans lequel ledit microorganisme producteur de cellulase extracellulaire et/ou intracellulaire est choisi dans le groupe constitué de procaryote, de bactérie, d'archée et d'eucaryote et de champignons.

10. Procédé selon la revendication 1, dans lequel ladite au moins une souche de microorganisme est tolérante vis-à-vis d'un ou de plusieurs composé(s) produit(s) par un prétraitement de la biomasse, dans lequel ledit ou lesdits composé(s) est/sont choisi(s) dans le groupe constitué de furfural, d'acide acétique et autres impuretés.

11. Procédé selon la revendication 1, dans lequel ladite ou lesdites cellulase(s) est/sont au moins un élément choisi dans le groupe constitué d'endoglucanase, d'exoglucanase et de β-glucosidase, et d'hémicellulases et, éventuellement, de laccase.

12. Procédé selon la revendication 1, comprenant en outre la mesure de l'activité de cellulase et/ou d'hémicellulase de l'étape (i) et, en fonction de l'activité de l'enzyme, le passage à l'étape (ii).

13. Procédé selon la revendication 1, dans lequel ladite au moins une souche d'algue de l'étape (iii) métabolise ledit au moins un élément parmi le glucose, le cellobiose, le xylose, le mannose, le galactose, le rhamnose, l'arabinose ou d'autres sucres d'hémicellulose, et les résidus du glycérol.

14. Procédé selon la revendication 1, dans lequel ladite au moins une souche d'algue de l'étape (iii) utilise de l'acide acétique comme source de carbone.

15. Procédé selon la revendication 1, dans lequel ladite au moins une souche d'algue utilise la respiration, lorsque l'étape (iii) est dans des conditions d'aérobie et d'hétérotrophie.

16. Procédé selon la revendication 1, comprenant, en outre, (iv) la récupération dudit ou desdits acide(s) gras de ladite au moins une souche d'algue.

17. Procédé selon la revendication 1, dans lequel l'étape (iii) comprend en outre la culture et la croissance de ladite au moins une souche d'algue dans des conditions permettant une production extracellulaire et/ou intracellulaire d'au moins un composé choisi dans le groupe constitué d'acides gras, d'hydrocarbures, de protéines, de pigments, de sucres, tels que les polysaccharides et les monosaccharides, et de glycérol.

18. Procédé selon la revendication 16, comprenant en outre, après l'étape (iv), la transestérification directe de cellules de ladite au moins une souche d'algue pour produire des acides gras pour le carburant biodiesel.

19. Procédé selon la revendication 18, dans lequel ladite transestérification directe comprend la rupture des cellules d'algue, la libération d'acides gras et la transestérification par l'intermédiaire d'un procédé acide ou de base avec du méthanol ou de l'éthanol pour produire un carburant biodiesel.

20. Procédé selon la revendication 1, dans lequel ladite au moins une souche d'algue est conçue pour utiliser des résidus de glycérol, comme source de carbone, produit par la réaction de transestérification sans prétraitement ni raffinage afin de produire des acides gras pour production de biodiesel.
